# EUROPÄISCHE PATENTSCHRIFT

(11) **EP 4 284 290 B1**
(45) Veröffentlichungstag und Bekanntmachung des Hinweises auf die Patenterteilung: **05.08.2026**
(21) Anmeldenummer: 22709202.0
(22) Anmeldetag: 31.01.2022
(51) Int. Cl.: A61B 90/20, A61B 90/00, A61B 17/00, A61B 34/20, A61B 90/50

(54) **CHIRURGISCHES ASSISTENZSYSTEM MIT OPERATIONSMIKROSKOP UND KAMERA UND DARSTELLUNGSVERFAHREN**
SURGICAL ASSISTANCE SYSTEM HAVING SURGICAL MICROSCOPE AND CAMERA, AND DISPLAYING METHOD
SYSTÈME D'ASSISTANCE CHIRURGICAL À MICROSCOPE OPÉRATOIRE ET CAMÉRA ET PROCÉDÉ DE VISUALISATION

(30) Priorität: 01.02.2021 DE 102021102274
(43) Veröffentlichungstag der Anmeldung: 06.12.2023
(73) Patentinhaber: B. Braun New Ventures GmbH, 79110 Freiburg im Breisgau (DE)
(72) Erfinder: SARVESTANI, Amir, 79104 Freiburg (DE)
(74) Vertreter: Winter, Brandl - Partnerschaft mbB
(86) Internationale Anmeldenummer: PCT/EP2022/052202
(87) Internationale Veröffentlichungsnummer: WO 2022/162217

(56) Entgegenhaltungen:
- EP-A1- 3 449 859
- WO-A1-2020/054566
- DE-A1- 102018 110 795
- US-A1- 2020 242 755

## Beschreibung

### Technisches Gebiet

Die vorliegende Offenbarung betrifft ein chirurgisches Assistenzsystem mit einem Operationsmikroskop/chirurgischen Mikroskop zur Verwendung bei einem chirurgischen Eingriff bei einem Patienten. Das Operationsmikroskop weist dabei einen beweglichen Mikroskopkopf mit einem Gehäuse und ein, insbesondere in dem Gehäuse vorgesehenen, optisches System auf, das dafür angepasst ist, in Richtung einer optischen Mikroskopachse eine optische Vergrößerung eines anvisierten Bereichs (eines Eingriffsbereich), insbesondere in einer Entfernung zwischen 5cm bis 80cm, besonders bevorzugt zwischen 20cm und 60cm, bereitzustellen und mittels einer Mikroskopaufnahmeeinheit, die insbesondere einen Sensor aufweist, eine (digitale) Mikroskopaufnahme zu erstellen und digital/computerlesbar bereitzustellen. Ferner weist das Operationsmikroskop einen an einer Basis vorgesehenen artikulierbaren/beweglichen Mikroskoparm auf, an dem der bewegliche Mikroskopkopf angeordnet, insbesondere gelagert, ist und der dafür angepasst ist, eine Position und/oder Orientierung des Mikroskopkopfes im Raum einzustellen. Das chirurgische Assistenzsystem weist ferner zumindest eine Darstellungsvorrichtung zur Anzeige eines visuellen Inhalts sowie eine Steuereinheit auf, die dafür angepasst ist, die Mikroskopaufnahme zu verarbeiten und die Darstellungsvorrichtung für eine Darstellung entsprechend anzusteuern. Daneben betrifft die vorliegende Offenbarung ein Aufnahmedarstellungsverfahren, ein Speichermedium sowie einen Sterilraum gemäß den Oberbegriffen der nebengeordneten Ansprüche.

### Stand der Technik

Chirurgische Mikroskope bzw. Operationsmikroskope gehören insbesondere in der Neurochirurgie, der Wirbelsäulenchirurgie und der Mikrochirurgie zu den Standardgeräten, welche bei einem chirurgischen Eingriff zum Einsatz kommen. Diese verwendeten Operationsmikroskope verfügen dabei üblicherweise über ein binokulares optisches System, mit dem ein Chirurg das Mikroskopbild/das mikroskopische Bild/die Mikroskopaufnahme unmittelbar optisch vergrößert sehen kann, oder über eine Darstellungsvorrichtung/ein externes Anzeigegerät, wie etwa einen OP-Monitor, zur visuellen Darstellung des (digital) aufgenommenen Mikroskopbildes vergrößert ausgeben lassen kann. Durch die Binokulare bzw. den OP-Monitor sieht der Chirurg in beiden Fällen jedoch nur die Darstellung des Mikroskopbildes eines anvisierten Eingriffsbereiches mit sehr hoher Vergrößerung. In Folge ist der Chirurg nicht oder zumindest nur schwer in der Lage, einen (das Mikroskopbild) umgebenden Operationsbereich/Operationsfeld/Eingriffsbereich einzusehen, um etwa seine Hände oder medizinische Instrumente, welche er beim chirurgischen Eingriff verwendet, zu orten. Lediglich eine Spitze eines chirurgischen Instruments gelangt bei entsprechender Positionierung in ein Sichtfeld des Operationsmikroskops.

Eine Sicht auf einen umgebenden Bereich des Mikroskopbilds ist jedoch äußerst wichtig, um etwa Schäden außerhalb des eigentlichen Eingriffsbereichs zu vermeiden. Um den umgebenden Bereich zu sehen, muss der Chirurg also vom binokularen System bzw. von der Darstellungsvorrichtung des Mikroskops wegschauen, schnell die Umgebung des durch das Operationsmikroskop anvisierten Bereichs visuell erfassen und sich hiernach wieder dem Mikroskopbild zuwenden, was zu einer enormen Belastung bei einem Eingriff führt. Ein ständiger Wechsel zwischen verschiedenen Blickrichtungen auf die Beiden Ansichtsmodalitäten sowie ein ständiges Korrelieren im Kopf des Chirurgen wirken ermüdend und führen oft zu ungewollten Fehlern.

Auch muss eine Anordnung des Operationsmikroskops in einem Operationssaal so erfolgen, dass insbesondere der leitende Chirurg einerseits das Operationsmikroskop gut handhaben und in dieses hineinschauen oder ein gutes Blickfeld auf einen OP-Monitor hat, und andererseits eine gute Sicht auf das Operationsfeld/den Operationsbereich/den Eingriffsbereich hat. Der OP-Monitor muss also so angeordnet werden, dass der Chirurg eine gute Sicht auf beide Modalitäten hat, was jedoch eine Flexibilität einer Anordnung im Operationssaal einschränkt. Auch ist für den Chirurgen eine direkte Sicht auf den Eingriffsbereich erschwert, da ein Bauvolumen eines Mikroskopkopfes des Mikroskops die direkte Sicht auf den Patienten meist blockiert. Zudem ist diese Position und entsprechend gute Sicht nur dem Chirurgen vorbehalten, wohingegen andere OP-Beteiligte an anderen Position im Operationssaal eine erschwerte Sicht auf einerseits das durch den OP-Monitor angezeigte Mikroskopbild und andererseits auf den Eingriffsbereich haben. Ein Medizinisches Personal ist bei ungünstigem Standpunkt somit genötigt, ständig den Kopf zu drehen und eine Blickrichtung wechseln.

Die US 20190290101 A1 offenbart beispielsweise ein Assistenzsystem mit einem Operationsmikroskop, das in Kombination mit einer tragbaren, an einem Kopf des Chirurgen vorgesehenen Visualisierungssystem verwendet wird. Durch dieses Visualisierungssystem wird der Chirurg je nach Blickwinkel seines Auges in die Lage versetzt, entweder ein Mikroskopbild oder nur eine Art LupenbildNergrößerungsansicht zu sehen. Hierdurch kann der Chirurg zwischen zwei Modalitäten wechseln, jedoch ist ein solches Visualisierungssystem mit Einschränkungen und Schwierigkeiten verbunden. Das Visualisierungssystem weist aufgrund der integrierten Optik ein hohes Gewicht auf, ist teuer in der Produktion und zudem fehleranfällig, da stets ein Strahlengang zum Auge des Chirurgen korrekt verlaufen muss. Auch verliert der Chirurg durch das Visualisierungssystem eine Wahrnehmung der Umgebung.

Die US 9,936,863 B2 etwa offenbart ein System mit einem Operationsmikroskop, das zusätzliche Kameras an einer Wundspreizvorrichtung am Patienten aufweist, um dem Chirurgen insbesondere eine zentrale mikroskopische Aufnahme des Operationsmikroskops sowie umfänglich Aufnahmen der an der Wundspreizvorrichtung vorgesehenen zusätzlichen Kameras bereitzustellen. Ein solches System hat jedoch den Nachteil, dass einerseits eine Wundspreizvorrichtung mit Kameras am Patienten vorgesehen werden muss und zudem eine Umgebung um die Eingriffsstelle herum nicht optisch erfasst wird, die jedoch dem Chirurgen entscheidende Informationen liefert.

Die EP 3 449 859 A1 offenbart ein Steuergerät, ein Steuerverfahren und ein chirurgisches System. An einem Gehäuse des Mikroskops oder an einer Decke ist eine Kamera angeordnet, um den Mikroskoparm zu erfassen.

Die DE 10 2018 110 795 A1 offenbart ein Mikroskop mit einem intelligenten Gerät, welches auf ein Objektfeld gerichtet ist.

Die US 2020/242755 A1 offenbart ein Abbildungssystem mit einer beweglichen optischen Einheit.

### Zusammenfassung der Offenbarung

Es ist daher die Aufgabe der vorliegenden Offenbarung, die Nachteile aus dem Stand der Technik zu vermeiden oder wenigstens zu mindern und insbesondere ein chirurgisches Assistenzsystem, ein (Aufnahme-)Darstellungsverfahren, ein Speichermedium sowie einen Sterilraum zur Verfügung zu stellen, welches/welcher OP Beteiligten/medizinischem Personal, insbesondere einem (leitenden) Chirurgen, mit einem Blick eine zentrale intuitive und unterstützende Verschmelzung von Informationen einer (vergrößerten) Mikroskopaufnahme / eines Mikroskopbild eines anvisierten Bereichs einer Eingriffsstelle sowie einer Darstellung einer Umgebung um den anvisierten Bereich der Eingriffsstelle herum bereitstellt. Zudem soll eine Hand-Augen-Koordination verbessert sowie ein sicherer Zugang zum Eingriffsbereich/Operationsbereich/Operationsfeld gewährleistet sein, um insbesondere Gewebeschäden durch unachtsame und unbeabsichtigte Handlungen als auch eine Dauer eines chirurgischen Eingriffs vermeiden oder zumindest zu minimieren. Insbesondere soll dem Chirurgen eine für den Eingriff notwendige Bewegung seines Kopfs sowie eine gedankliche Belastung durch Verknüpfung von Informationen der Mikroskopaufnahme und der Umgebung minimiert werden.

Die Aufgaben werden hinsichtlich eines gattungsgemäßen chirurgischen Assistenzsystems durch die Merkmale des Anspruchs 1, hinsichtlich eines gattungsgemäßen Aufnahmedarstellungsverfahren durch die Merkmale des Anspruchs 11, hinsichtlich eines gattungsgemäßen computerlesbaren Speichermediums durch die Merkmale des Anspruchs 12 und hinsichtlich eines gattungsgemäßen Sterilraums durch die Merkmale des Anspruchs 13 gelöst.

Grundsätzlich ist das Operationsmikroskop, insbesondere der Mikroskopkopf, mit einem Kamerasystem mit zumindest einer zusätzlichen Kamera ausgestattet. Die Kamera ist am Mikroskopkopf, insbesondere am Gehäuse des Mikroskop-Optiksystems, angeordnet, insbesondere fix/starr befestigt. Damit weist die optische Mikroskopachse in ähnliche Blickrichtung wie die optische Kameraachse. So sind beide Aufnahmen, die Mikroskopaufnahme und die Kameraaufnahme insbesondere korreliert. Die zusätzliche Umgebungskamera erzeugt Aufnahmen mit im Vergleich zum Operationsmikroskop geringerer Vergrößerung bzw. vorzugsweise Weitwinkelaufnahmen/Weitwinkelbilder mit ähnlicher Blickrichtung wie das optische System des Operationsmikroskops. Die Umgebungskamera oder eine weitere Kamera des Kamerasystems kann auch so an dem Mikroskopkopf angeordnet sein, dass diese innerhalb des Mikroskopkopfs, insbesondere innerhalb des Gehäuses, angeordnet bzw. integriert ist.

Beide Aufnahmen, sowohl die Mikroskopaufnahme durch das Operationsmikroskop (bzw. das optische System) als auch die Umgebungsaufnahme durch die Umgebungskamera werden durch die Steuereinheit verarbeitet und es wird eine Kombinationsdarstellung mit beiden Aufnahmen erzeugt. Diese (digital erzeugte) Kombinationsdarstellung, in der Informationen zu beiden Aufnahmen visuell vorliegen, werden dann zentral durch die Darstellungsvorrichtung ausgegeben.

Das Assistenzsystem ermöglicht es somit dem Nutzer, insbesondere dem Chirurgen, sowohl vergrößert das Innere des Eingriffsbereichs als auch für einen Überblick die Umgebung/das Äußere des Eingriffsbereichs visuell zu erfassen, ohne dass er seine Kopfposition oder seine Blickrichtung ändern muss. Die zentrale Darstellung verbessert insbesondere eine Koordination, wirkt einer Ermüdung entgegen und verkürzt einen Eingriff. Auch wird eine Sicherheit eines Patienten erhöht, da dem Chirurgen eine bessere Übersicht bereitgestellt wird.

Mit anderen Worten weist das Operationsmikroskop, insbesondere der Mikroskopkopf, ein Kamerasystem mit zumindest einer Umgebungskamera auf, wobei die Umgebungskamera an dem Mikroskopkopf angeordnet bzw. mit dem Mikroskopkopf verbunden ist. Insbesondere ist die Umgebungskamera an dem Gehäuse befestigt. Vorzugsweise ist die Umgebungskamera starr an dem Mikroskopkopf, insbesondere dem Gehäuse, fixiert, so dass eine Ausrichtung gegenüber dem Mikroskopkopf sowie dem optischen System stets konstant bleibt. Die Umgebungskamera ist dabei derart angepasst, insbesondere über eine Ausrichtung ihrer optischen Kameraachse und/oder über ihre Kameraoptik, dass ein Sichtfeld der Umgebungskamera, insbesondere ab einer vorbestimmten Entfernung, vorzugsweise ab einer Entfernung von 5cm zu einer Front der Kamera oder zu einer Seite des Mikroskopkopfs, das Sichtfeld des optischen Systems des Operationsmikroskops mit einschließt/umfasst/mit aufweist/beinhaltet, um durch die Umgebungskamera sowohl den von dem optischen System anvisierten Bereich als auch eine Umgebung um den anvisierten Bereich herum zu erfassen und eine Umgebungsaufnahme digital/computerlesbar bereitzustellen. Die Steuervorrichtung ist ferner dafür angepasst, die Umgebungsaufnahme zu verarbeiten und eine Kombinationsdarstellung mit der Mikroskopaufnahme und der Umgebungsaufnahme zu erzeugen und durch die Darstellungsvorrichtung visuell auszugeben.

Eine Kombinationsdarstellung bedeutet im vorliegenden Fall, dass sowohl die Informationen der Mikroskopaufnahme als auch der Umgebungsaufnahme in einer einzigen Darstellung, der Kombinationsdarstellung, zentral widergegeben werden. Mit anderen Worten ist in der Kombinationsdarstellung die Mikroskopaufnahme und die Umgebungsaufnahme, gegebenenfalls durch eine Bildverarbeitung weiterverarbeitet, zu sehen.

Insbesondere hat also die Umgebungskamera eine ähnliche oder parallele Blickrichtung wie das optische System des Operationsmikroskops. So wird neben dem anvisierten Bereich zudem eine Umgebung um diesen anvisierten Bereich optisch erfasst. Man kann auch sagen, dass die optischen Achsen von dem optischen System und der Umgebungskamera ähnlich, insbesondere parallel oder im Wesentlichen parallel zueinander ausgerichtet sind. An dieser Stelle wird angemerkt, dass eine optische Erfassung insbesondere nicht nur auf den sichtbaren Bereich des Lichts beschränkt sein muss, sondern sie kann auch weitere Frequenzen unterhalb und oberhalb des sichtbaren Bereichs umfassen. Beispielsweise kann durch die Umgebungskamera auch eine Infrarotaufnahme als Umgebungsaufnahme aufgenommen, durch die Steuereinheit verarbeitet und entsprechend ausgegeben werden. Vorzugsweise kann die Umgebungskamera, insbesondere die Umgebungskamera und die Steuereinheit, oder eine weitere Kamera des Kamerasystems dafür angepasst sein, ein 3D Bild und/oder eine sogenannte Depth Map aufzunehmen bzw. zu erzeugen. Auf diese Weise erhält man nicht nur ein zweidimensionales Bild, sondern auch eine 3D-Struktur der Umgebung. Eine Depth Map weist Informationen zu Distanzen von einem Blickpunkt zu einer Oberfläche hin auf, so dass so eine 3D-Struktur erfasst wird.

Der Begriff Sichtfeld definiert einen Bereich im Bildwinkel des optischen Systems bzw. der Umgebungskamera, innerhalb dessen eine Aufnahme erfolgen kann. Es ist sozusagen das Bild/die Aufnahme, das/die auf die Aufnahmeeinheit bzw. einen Sensor fällt, begrenzt durch die Ränder. Man kann dies etwa mit einem Kegel vergleichen, der koaxial zur optischen Achse ausgerichtet ist und dessen Spitze an einer Front der Optik angeordnet ist. Alle "Objekte" in dem Innenvolumen des Kegels können aufgenommen werden. Ein Öffnungswinkel des Kegels definiert dabei den möglichen Aufnahmebereich. Vorliegend liegt also bildlich gesehen der Kegel des Mikroskops in dem Kegel (innerhalb) der Umgebungskamera. So kann die Umgebungskamera einerseits den anvisierten Bereich erfassen, da der anvisierte Bereich ja auch innerhalb des Kegels der Umgebungskamera liegt, und zudem eine Umgebung um diesen Bereich herum. Während die Umgebungskamera einen weiten Öffnungswinkel (des Kegels) mit entsprechend kleiner Vergrößerung aufweist, hat das optische System des Mikroskops einen sehr engen Öffnungswinkel mit entsprechend hoher Vergrößerung.

Das Assistenzsystem weist ferner eine Speichereinheit mit darin gespeicherten präoperativen 3D-Aufnahmedaten, insbesondere MRT-Aufnahmedaten und/oder CT-Aufnahmedaten, auf und das Assistenzsystem, insbesondere die Steuereinheit, ist dafür angepasst, mittels dem Kamerasystem, insbesondere mit nur der Umgebungskamera, eine (dreidimensionale) 3D-Struktur des Patienten, insbesondere eines Kopfes, besonders bevorzugt eines Gesichts, des Patienten, räumlich zu erfassen und die erfasste 3D-Struktur mit den 3D-Aufnahmedaten zu korrelieren, um den Patienten, insbesondere gegenüber dem Operationsmikroskop, besonders bevorzugt dem Mikroskopkopf, zu registrieren. Mit anderen Worten kann insbesondere die Steuereinheit dafür angepasst sein, auf Basis von hinterlegten 3D-Aufnahmedaten des Patienten, insbesondere von hinterlegten Daten einer Magnetresonanztomografie (MRT) oder Computertomografie (CT), eine aufgenommene 3D-Struktur mit den 3D-Aufnahmedaten zu korrelieren, um den Patienten zu registrieren. Auf diese Weise wird ein lokales Koordinatensystem des Patienten mit dem lokalen Koordinatensystem des Operationsmikroskops, insbesondere des Mikroskopkopfes, über eine Transformationsmatrix verknüpft. So können die 3D-Aufnahmedaten mit dem Patienten in geometrischer Relation gesetzt werden bzw. mit dem Mikroskopkopf verknüpft werden. Über die Umgebungskamera kann so in einfacher Weise der Patient registriert werden. Wird der Mikroskopkopf bewegt, so kann die Bewegung des Mikroskopkopfs erfasst/getrackt und mit den 3D-Aufnahmedaten entsprechend korreliert werden. Eine weitere Funktion des Assistenzsystems mit der Umgebungskamera ist also die Registrierung des Patienten bei Verwendung eines chirurgischen Navigationssystems. Mit noch anderen Worten kann die zusätzliche Umgebungskamera zur Registrierung des Patienten mittels Mustererkennung verwendet werden. Die 3D-Oberfläche des Patienten, beispielsweise das Gesicht, wird extrahiert und mit der 3D-Oberfläche von 3D-Aufnahmedaten/eines präoperativen 3D-Datensatzes, wie etwa CT-Aufnahmedaten oder MRT-Aufnahmedaten, korreliert. Die Umgebungskamera erzeugt also ein 3D-Bild einer Patientenoberfläche, beispielsweise des Gesichts, das mit präoperativen 3D-Aufnahmedaten, beispielsweise MRT-Aufnahmedaten, korreliert werden kann, wofür die Steuereinheit entsprechend angepasst ist. Externe Referenzen für eine Korrelation können insbesondere starre Körper und/oder optische Muster und/oder charakteristische Landmarken sein. Die Umgebungskamera wird auf den Patienten auf den Bereich ausgerichtet, der für die Registrierung verwendet wird, beispielsweise das Gesicht. Der Begriff 3D definiert dabei, dass die Strukturen, Aufnahmedaten bzw. Oberflächen des Patienten räumlich, also dreidimensional vorliegen. Der Körper des Patienten oder zumindest ein Teilbereich des Körpers mit räumlicher Ausdehnung kann in einem dreidimensionalen Raum mit etwa einem kartesischen Koordinatensystem (X, Y, Z) digital als Aufnahmedaten vorliegen.

Vorteilhafte Ausführungen sind in den Unteransprüchen beansprucht und werden nachfolgend erläutert.

Gemäß einer weitere Ausführungsform kann zusätzlich oder alternativ zu der Registrierung über die 3D-Struktur eine Registrierung über zumindest einen an dem Patienten angebrachten Marker erfolgen. Die Steuereinheit ist dabei angepasst, über eine erfasste vordefinierte Lage des zumindest einen Markers den Patienten gegenüber dem Operationsmikroskop, insbesondere dem Mikroskopkopf, zu registrieren. Die Registrierung kann also mit Markern oder ohne Marker (Markerlos) mit Hilfe einer Bildverarbeitungstechnologie erfolgen. Der Begriff Lage definiert sowohl eine Position als auch eine Orientierung.

Vorzugswiese kann zur Erfassung einer 3D-Struktur das Kamerasystem eine 3D-Kamera und/oder eine 2D-Kamera aufweisen, insbesondere aus nur der Umgebungskamera als einzige 2D-Kamera oder 3D-Kamera des Kamerasystems bestehen. Im Falle der 2D-Kamera kann diese über einen interessierenden Bereich des Patienten bewegt werden, um verschiedene Ansichten des interessierenden Bereichs zu erfassen. Die Steuereinheit ist dann dafür angepasst, mittels Bildanalyse (Machine Vision) aus den verschiedenen aufgenommenen Ansichten eine 3D-Struktur zu berechnen. Zur Erstellung einer 3D-Oberfläche/3D-Struktur wird also eine 3D-Kamera verwendet oder eine 2D-Kamera, die über den Patienten bewegt wird und Bilder aus verschiedenen Perspektiven erzeugt. Die so erzeugte 3D-Struktur/3D-Oberfläche kann dann über die Steuereinheit insbesondere mit präoperativen 3D-Aufnahmedaten korreliert werden.

Gemäß einem Aspekt der Offenbarung kann das Assistenzsystem insbesondere zumindest einen Sensor aufweisen und dafür angepasst sein, eine Position, insbesondere eine Lage, des Mikroskopkopfs, zu erfassen und der Steuereinheit als Positionsdaten bzw. Lagedaten bereitzustellen. Die Steuereinheit kann vorzugsweise ihrerseits dafür angepasst sein, die Position, insbesondere die Lage, des Mikroskopkopfs, bei Registrierung, in einer Speichereinheit als Registrierungsposition sowie eine zugehörige Umgebungsaufnahme, insbesondere eine erfasste 3D-Struktur, zu speichern. Bei erneuter Registrierung in der hinterlegten Registrierungsposition bzw. Lage kann die Steuereinheit bestimmen, dass eine Abweichung der Registrierung vorliegt, wenn eine Überlagerung der hinterlegten 3D-Struktur und der erneut erfassten 3D-Struktur, insbesondere in Teilbereichen der Überlagerung, die sich während eines Eingriffs nicht ändern (wie etwa Abdeckungen oder Wundränder/Schnittkanten), über einen vorbestimmten hinaus Grenzwert abweicht. Insbesondere kann die Steuereinheit dafür angepasst sein, einen Betrag einer Abweichung zu berechnen. Beispielsweise kann eine Menge an charakteristisch vordefinierter Punkte bestimmt werden und ein Abstand zwischen den korrespondierenden Punkten der hinterlegten 3D-Struktur und der neu erfassten 3D-Struktur aufsummiert werden. Wenn die Summe der Abstände einen Grenzwert übersteigt, kann bestimmt werden, dass eine Abweichung vorliegt. Vorzugsweise kann die Steuereinheit dafür angepasst sein, die Registrierung um einen Offset, insbesondere um einen Offset einer Translation im Raum und/oder einer Rotation um eine Achse, automatisch zu korrigieren. Mit anderen Worten kann das Assistenzsystem vorzugsweise dafür angepasst sein, eine Abweichung zwischen zwei Umgebungsaufnahmen (aus der gleichen Lage) zu erkennen, einen Offset/Versatz/Abweichung zu berechnen und um den vorbestimmten Offset zu korrigieren. Insbesondere kann die Steuereinheit einen Nutzer bei Vorliegen einer Abweichung informieren, beispielsweise visuell über die Darstellungsvorrichtung und/oder akustisch über ein Audiogerät. Insbesondere kann ein Alarmsignal über das Assistenzsystem ausgegeben werden. Insbesondere kann also das Assistenzsystem mittels der Umgebungskamera und der angepassten Steuereinheit eine automatische Erkennung und Korrektur von Ungenauigkeiten in der Navigation durch etwa unbeabsichtigte Bewegungen eines Patiententrackers durchführen. Die Umgebungskamera und das optische System des Operationsmikroskops sind geometrisch korreliert. Bei oder nach der initialen Registrierung nimmt die Umgebungskamera ein Bild des Eingriffsbereichs von einer bestimmten Position (Registrierungsposition) oder Lage aus auf. Diese Position bzw. Lage wird als relative Transformation (Transformationsmatrix) eines Patiententrackers zu einem Mikroskoptracker berechnet. Um die Genauigkeit zu überprüfen, kann der Mikroskopkopf während der Operation jederzeit in diese Registrierungsposition (zurück-)bewegt werden, um erneut ein Bild (Umgebungsaufnahme) aufzunehmen. Beide Bilder - das von der ursprünglichen Registrierung und das von der Genauigkeitsüberprüfung (die erneute Umgebungsaufnahme) werden überlagert. Liegt keine Ungenauigkeit bzw. Abweichung vor, stimmen die Bilder in allen speziellen Bildausschnitten/Bildbereichen überein, die während des Eingriffs nicht verändert werden (wie etwa Abdeckungen oder Wundränder/Schnittkanten). Liegt hingegen eine Abweichung bzw. Ungenauigkeit vor, haben die beiden Aufnahmen/Bilder einen Versatz zueinander. Insbesondere kann der Nutzer über die Abweichung informiert werden. Durch eine Berechnung eines Offsets kann der Chirurg zudem auch über die Höhe der Abweichung informiert werden. Zusätzlich kann vorzugsweise die Ungenauigkeit automatisch durch die Steuereinheit korrigiert werden, indem die Registrierung mit dem ermittelten Offset angepasst wird.

Gemäß einem weiteren Aspekt der Offenbarung kann alternativ oder zusätzlich zu der zu hinterlegenden Registrierungsposition auch eine geometrische Position, insbesondere Lage des Mikroskopkopfs, vorbestimmt sein, in welche der Mikroskopkopf bewegt wird, um von dort aus die Umgebungsaufnahme, insbesondere über die Erfassung eines Eingriffsbereichs oder einer Erfassung eines Gesichts des Patienten, durchzuführen. Die vorstehenden Merkmale der Konfiguration des Assistenzsystems zur Berechnung der Abweichung sind auch in diesem Aspekt wiederzufinden.

Insbesondere kann das Operationsmikroskop zumindest einen Aktuator aufweisen, um den beweglichen Mikroskoparm und den beweglichen Mikroskopkopf aktiv zu bewegen und zu steuern und die Steuervorrichtung kann ferner dafür angepasst sein, den Aktuator anzusteuern, um den Mikroskopkopf in eine vorbestimme Position und/oder Orientierung im Raum zu bewegen. Auf diese Weise kann der Mikroskopkopf automatisch, ohne manuelle Handhabung, insbesondere in eine vorbestimmte Lage (Position und Orientierung) "gefahren" werden. Auf diese Weise kann etwa die vorstehend erläuterte Registrierungsposition eineindeutig wiederholungsgenau eingenommen werden oder es kann eine gespeicherte Ansicht durch das Operationsmikroskop wieder "aufgerufen" werden.

Gemäß einem ggf. unabhängig beanspruchbaren Aspekt der Erfindung kann die Umgebungskamera und/oder die Aufnahmeeinheit einen hochauflösenden Sensor mit vorzugsweise mindestens 35 Megapixel aufweisen, und die Steuereinheit dafür angepasst sein, einen, insbesondere zentralen, Bereich des Sensors für eine Aufnahme digital zu vergrößern, während für einen anderen Bereich des Sensors eine geringere Vergrößerung verwendet wird.

Gemäß einem weiteren Aspekt der Offenbarung kann die Steuereinheit dafür angepasst sein, insbesondere initial, eine Umgebungsaufnahme zu erstellen, in der Umgebungsaufnahme zumindest eine charakteristische Landmarke zu extrahieren und anhand einer erfassten Lage des Mikroskopkopfs (mit definierter geometrischer Relation von Mikroskopkopf zu Umgebungskamera) eine Position, insbesondere Lage, der extrahierten Landmarke relativ zu dem Mikroskopkopf und damit der Landmarke im Raum zu einem Referenzkoordinatensystem zu bestimmen und die charakteristische Landmarke der Umgebungsaufnahme sowie die zugehörige bestimmte Position bzw. Lage als Referenzwert in der Speichereinheit zu hinterlegen, wobei die Steuereinheit ferner dafür angepasst ist, kontinuierlich in einer aktuellen Umgebungsaufnahme diese extrahierte Landmarke zu erkennen und kontinuierlich eine aktuelle Position, insbesondere aktuelle Lage, der Landmarke im Raum zu bestimmen, und bei einer Abweichung zwischen der hinterlegten Position bzw. Lage und der aktuellen Position bzw. Lage zu bestimmen, dass eine Abweichung der Registrierung vorliegt. Insbesondere gibt die Steuereinheit dann über die Darstellungsvorrichtung eine entsprechende Meldung aus. Mit anderen Worten kann also in einer Ausführungsform anstatt einer Erkennung von Abweichungen in einer Registrierungsposition auch ein Verfahren bzw. eine entsprechend angepasste Steuereinheit verwendet werden, um eine Abweichung der Registrierung in nicht vorbestimmten Positionen oder Lagen zu bestimmen. Hierbei wird ein Teilbereich des von der Umgebungskamera beobachteten bzw. erfassten Bereiches, der sich während des Eingriffs nicht ändert, herangezogen. In diesem Bereich werden verfolgbare Landmarken mittels Methoden der Bildverarbeitung extrahiert. Landmarken können initial zu Beginn des Eingriffs bzw. der Prozedur, oder kontinuierlich während des Eingriffs gefunden bzw. bestimmt werden. Die Landmarken müssen so geartet sein, dass diese unter verschiedenen Positionen und/oder Orientierungen des Mikroskopkopfs mittels der Umgebungskamera erkennbar sind. Die Position jeder im Sichtfeld der Umgebungskamera liegenden Landmarke wird insbesondere mittels Methoden der Bildverarbeitung kontinuierlich relativ zu der Umgebungskamera bestimmt. Aufgrund der bekannten geometrischen Relation bzw. Lage von der Umgebungskamera relativ zu dem Mikroskopkopf und aufgrund einer messbaren Lage des Mikroskopkopfs, ist damit auch die Lage der beobachteten Landmarken bekannt bzw. kann durch die Steuereinheit zurückberechnet bzw. bestimmt werden. Die so bestimmte Landmarkenposition kann dann mit der erwarteten Landmarkenposition bzw. -lage (Soll-Position) verglichen werden. Wird hier eine Abweichung beobachtet, so kann von einer Abweichung der Registrierung ausgegangen werden. Die erwartete Landmarkenposition wird hierzu insbesondere initial bei der ersten Beobachtung bzw. Erfassung einer Landmarke im System bzw. in der Speichereinheit hinterlegt, um sie bei weiteren Erfassungen der Landmarke als Referenzwert heranziehen zu können.

Gemäß einem weiteren Aspekt der Offenbarung kann die Steuereinheit dafür angepasst sein, mittels Methoden der Bildverarbeitung eine Position, insbesondere eine Lage, einer Landmarke relativ zu der Umgebungskamera zu bestimmen und mit einer bekannten Lage der Landmarke zu vergleichen, insbesondere mit einer erfassten Lage der Landmarke durch eine externe Kamera des Navigationssystems und/oder einer erfassten Lage durch das optische System und der Aufnahmeeinheit des Mikroskops, und unter Verwendung von Minimierungsverfahren des überbestimmten Systems eine Registrierung zu verbessern und/oder einen Fehler in Kamerasystemen zu minimieren. Mit anderen Worten kann gemäß einer Ausführungsform die bestimmte Position bzw. Lage der beobachteten bzw. charakteristischen Landmarken relativ zu der Umgebungskamera auch zur Verbesserung einer Genauigkeit des Navigationssystems bzw. einer Registrierung herangezogen werden. Hierzu wird analog insbesondere die Annahme gemacht, dass die Lage des Mikroskopkopfes, die Lage der Umgebungskamera und die Lage der Landmarken bekannt oder bestimmbar ist. Wird (durch die Steuereinheit) die Position, insbesondere Lage, der Landmarken mittels Methoden der Bildverarbeitung relativ zur Umgebungskamera bestimmt, so ergibt sich ein weiterer Weg, die Position der Landmarken über die Position, insbesondere Lage, des Mikroskops bzw. Mikroskopkopfs zu bestimmen. Aufgrund von Ungenauigkeiten in Kamerasystemen oder Registrierung werden bestimmte Landmarkenposition sich zwischen den beiden Wegen unterscheiden. Aus mathematischer Sicht ergibt sich ein überbestimmtes System. Unter Verwendung von Minimierungsverfahren kann diese Überbestimmtheit verwendet werden, um Fehler in Kamerasystemen und/oder in einer Registrierung des Navigationssystems kontinuierlich zu verbessern und somit die Genauigkeit des Gesamtsystems zu steigern.

Gemäß einem weiteren Aspekt der Offenbarung kann im Falle eines aktiv bewegbaren Mikroskopkopfs, das Assistenzsystem eine Eingabeeinheit, insbesondere einen Touchscreen, aufweisen, um in der Umgebungsaufnahme der Umgebungskamera einen Fokuspunkt auszuwählen. Die Steuereinheit kann wiederum dafür angepasst sein, auf Basis des gewählten Fokuspunkts über den zumindest einen Aktuator aktiv den Mikroskoparm und den Mikroskopkopf so zu steuern und zu bewegen, dass die optische Mikroskopachse, insbesondere in einem vorbestimmten zugehörigen Abstand und/oder in einem vorbestimmten Aufnahmewinkel, auf den ausgewählten Fokuspunkt bei dem Patienten ausgerichtet wird. So wird ein intuitives, automatisch justierbares Operationsmikroskop bereitgestellt, das es dem Chirurgen erlaubt, mit nur einer Berührung auf einen ausgewählten Punkt in der Umgebungsaufnahme das Mikroskop für eine passende Fokussierung auszurichten. Eine zusätzliche Funktion des Assistenzsystems mit der Umgebungskamera ist also, ein robotergeführtes Operationsmikroskop in ein Zielfeld zu bewegen, indem ein Zielpunkt/fokuspunkt in der Umgebungsaufnahme der Umgebungskamera definiert wird und das Mikroskop so bewegt wird, dass der Fokuspunkt im Fokus der Mikroskopaufnahme bzw. des optischen Systems des Operationsmikroskops liegt.

Gemäß einem weiteren Aspekt der Offenbarung kann die Steuereinheit dafür angepasst sein, mittels Bildanalyse der Umgebungsaufnahme und/oder einem an dem Mikroskopkopf vorgesehenen Sensor, ein Objekt im Bereich des Mikroskopkopfs sowie eine Distanz des beweglichen Mikroskopkopfs zu diesem Objekt zu erkennen. Bei einer möglichen Kollision mit diesem Objekt, insbesondere bei Unterschreitung einer vorbestimmten Distanz, ist das Assistenzsystem, insbesondere die Steuereinheit dafür angepasst, ein Alarmsignal, insbesondere einen Alarmton über ein Audiogerät und/oder eine Alarmanzeige über die Darstellungsvorrichtung auszugeben und/oder zumindest einen Bewegungsfreiheitsgrad des Mikroskopkopfs und/oder des Mikroskoparms zu beschränken, insbesondere insgesamt eine Bewegung des Mikroskopkopfs, insbesondere des gesamten Operationsmikroskops zu stoppen. Eine weitere Funktion des Assistenzsystems mit der zusätzlichen Umgebungskamera ist also, Kollisionen des Mikroskops mit Objekten zu verhindern, indem das Assistenzsystem einen Abstand zum Mikroskopkopf ermittelt und bei entsprechender Unterschreitung eines Abstandes eine Warnung visuell oder akustisch ausgibt oder im Falle eines robotergeführten Operationsmikroskops die Bewegung stoppt. Die Umgebungskamera/Übersichtskamera kann auch Kollisionen bei autonomen Bewegen des Mikroskopkopfs, der über den Mikroskoparm, insbesondere in Form eines Roboterarms, geführt wird, detektieren. Die Bewegung kann dann rechtzeitig gestoppt werden, etwa durch ein Brems- oder Verriegelungssystem. Auch wenn der Mikroskoparm bzw. Roboterarm manuell geführt wird, kann eine Kollision erkannt und eine weitere Bewegung unterbunden werden.

Vorzugsweise kann die Steuereinheit dafür angepasst sein, über das Kamerasystem, insbesondere über zumindest die Umgebungsaufnahme, insbesondere über Marker und/oder über charakteristische Merkmale des Patienten, den Patienten und/oder, insbesondere über Marker und/oder charakteristische Merkmale von medizinischen Instrumenten, ein medizinisches, insbesondere chirurgisches, Instrument und/oder den Mikroskopkopf lagekorrekt zu erfassen und insbesondere zu tracken. Durch die Umgebungskamera kann also kontinuierlich etwa ein medizinisches Instrument insbesondere in seiner Lage erfasst werden. Diese Lage des erfassten medizinischen Instruments kann insbesondere in den 3D-Aufnahmedaten wiedergegeben werden, indem eine virtuelle Position und/oder Orientierung schematisch dargestellt wird oder indem ein virtuelles Instrument in der entsprechenden Lage überlagert eingeblendet wird. Insbesondere kann eine relative Lage des Patienten und/oder medizinischen Instrumenten gegenüber dem Mikroskopkopf in den 3D-Aufnahmedaten lagekorrekt angezeigt werden. Weiter kann insbesondere auch ein erfasster Mikroskopkopf in den 3D-Aufnahmedaten als virtueller Mikroskopkopf angezeigt werden. Mit anderen Worten kann das Assistenzsystem mit der Umgebungskamera insbesondere dafür verwendet werden, eine Position, insbesondere eine Lage, des Patienten bei Verwendung des Operationsmikroskops zu verfolgen. Vorzugsweise können dafür externe Referenzen, wie starre Körper oder optische Muster oder eindeutige anatomische Orientierungspunkte, verwendet werden, um die Position, insbesondere Lage, des Patienten relativ zum Mikroskopkopf zu bestimmen bzw. zu lokalisieren. Dies ermöglicht, das Mikroskopbild korreliert mit den präoperativen 3D-Aufnahmedaten, wie etwa MRT-Aufnahmedaten, darzustellen. Die Umgebungskamera kann vorzugsweise auch dafür eingesetzt werden, chirurgische Instrumente zu verfolgen, die insbesondere mit Markern ausgestattet sind, um ihre Position, insbesondere Lage, im Mikroskopbild der präoperativen 3D-Aufnahmedaten zu überlagern. Eine weitere Funktion des Assistenzsystems mit der zusätzlichen Umgebungskamera besteht also darin, bei Verwendung eines chirurgischen Navigationssystems sie zum Tracken/zur Verfolgung des Patienten und/oder eines chirurgischen Instruments und/oder des Operationsmikroskops selbst vorzusehen. Es können etwa Marker im Sichtfeld der Umgebungskamera zum Tracking verwendet werden oder das Tracking erfolgt markerlos anhand eindeutiger Merkmale des Patienten oder der Instrumente. In diesem Fall kann die Umgebungskamera die typischerweise in chirurgischen Navigationssystemen verwendete externe Kamera ersetzen. Darüber hinaus kann die Umgebungskamera eine externe Kamera des chirurgischen Navigationssystems auch temporär ersetzen oder ergänzen, um Merkmale und Instrumente auch im Falle von temporär auftretenden Sichteinschränkungen (beispielsweise bedingt durch Überdeckungen) der externen Kamera zu erfassen und insbesondere lokalisieren zu können.

Gemäß einem weiteren Aspekt der Offenbarung können in einer Speichereinheit Daten, insbesondere geometrische Relationen, von zumindest einem medizinischen Instrument und zugehörige Benutzungshinweise hinterlegt/gespeichert sein. Die Steuereinheit kann ferner dafür angepasst sein, in der Umgebungsaufnahme anhand der gespeicherten Daten, insbesondere anhand der gespeicherten geometrischen Relation, ein chirurgisches Instrument zu erfassen und einem Nutzer die zugehörigen gespeicherten Benutzungshinweise über die Darstellungsvorrichtung und/oder ein Audiogerät auszugeben. Eine zusätzliche Funktion des Assistenzsystems mit Umgebungskamera ist also die Erkennung von verwendeten medizinischen, insbesondere chirurgischen, Instrumenten sowie eine Bereitstellung von zugehörigen Anwendungshinweisen für den Chirurgen.

Gemäß einer Ausführungsform kann die zumindest eine Darstellungsvorrichtung ein OP-Monitor und/oder ein Head-Mount-Display (HMD) und/oder ein binokulares System mit einblendbaren Daten sein. Im Falle des OP-Monitors als Darstellungsvorrichtung wird die Kombinationsdarstellung als Bild auf dem OP Monitor angezeigt. Im Falle, dass das Assistenzsystem ein Head-Mount-Display/eine Virtual-Reality-Brille als Darstellungsvorrichtung aufweist, kann die Kombinationsdarstellung über ein Display ausgegeben werden. Insbesondere kann die Kombinationsdarstellung, wenn das Operationsmikroskop und/oder die Umgebungskamera dafür angepasst ist eine 3D-Aufnahme zu erstellen, über das Head-Mount-Display die Kombinationsdarstellung dreidimensional ausgeben, so dass dem Chirurgen ein räumlicher Blick bereitgestellt wird. Im Falle eines binokularen Systems, durch welches der Chirurg mit beiden Augen hindurchsieht, können in einer Bildebene, etwa über ein Prisma und ein Display, die Umgebungsdaten mit eingeblendet werden. Dies stellt ein Augmented Reality System dar. Mit anderen Worten kann die zumindest eine Darstellungsvorrichtung ein (OP-)Monitor und/oder ein kopfgetragenes Display und/oder ein binokulares System sein.

Insbesondere kann die Steuereinheit dafür angepasst sein, als Kombinationsdarstellung eine Nebeneinanderdarstellung oder Überlagerungsdarstellung von der Mikroskopaufnahme und der Umgebungsaufnahme zu erstellen und über die Darstellungsvorrichtung auszugeben. Mit anderen Worten können die Mikroskopaufnahme und die Umgebungsaufnahme nebeneinander oder überlagert dargestellt werden. Bei überlagerter Darstellung kann insbesondere durch die Steuereinheit in einem zentralen Bereich der Kombinationsdarstellung die Mikroskopaufnahme (etwa ohne Transparenz) erstellt werden, wobei als Hintergrund um diese Mikroskopaufnahme herum die Umgebungsaufnahme dargestellt wird.

Gemäß einer weiteren Ausführungsform kann eine Vergrößerung des optischen Systems mindestens fünffach, insbesondere mindestens zehnfach, besonders bevorzugt mindestens vierzigfach sein. Vorzugsweise kann das optische System des Operationsmikroskops über eine Zoomfunktion verfügen. Weiter vorzugsweise kann eine Vergrößerung der Umgebungskamera maximal fünffach, insbesondere maximal einfach sein. Während das Operationsmikroskop mit dem optischen System und der Aufnahmeeinheit eine relativ gesehen hohe Vergrößerung liefert, ist die Umgebungskamera mit ihrer Optik dafür angepasst, eine Weitwinkelaufnahme zu erstellen und als Umgebungsaufnahme für eine Darstellung der Umgebung bereitzustellen.

Gemäß einem Aspekt der Offenbarung kann das Kamerasystem zusätzlich zu der Umgebungskamera zudem noch eine zweite Kamera, insbesondere zudem noch eine dritte Kamera, besonders bevorzugt zudem noch eine vierte Kamera aufweisen, um einen Sichtbereich um den Eingriffsbereich herum zu vergrößern. Beim Kamerasystem können also neben der Umgebungskamera noch zusätzliche Kameras eingesetzt werden, um das Sichtfeld außerhalb des Operationsfeldes zu vergrößern.

Insbesondere kann eine Brennweite einer Optik der Umgebungskamera minimal 10mm und/oder maximal 50mm betragen oder ein Blickwinkel mindestens 45°, vorzugsweise mindestens 70° betragen, um eine weitwinkelige Umgebungsaufnahme bereitzustellen.

Vorzugsweise kann die optische Mikroskopachse die optische Kameraachse schneiden oder in einem Abstand von maximal 10cm, besonders bevorzugt von maximal 5cm passieren. Insbesondere kann ein Winkel zwischen der optischen Mikroskopachse und der optischen Kameraachse minimal 2° und/oder maximal 15° sein. Alternativ liegen vorzugsweise die optische Mikroskopachse und die optische Kameraachse parallel zueinander.

Hinsichtlich eines (Aufnahme-)Darstellungsverfahren zur Anzeige von zwei unterschiedlichen Aufnahmen werden die Aufgaben und Ziele der vorliegenden Offenbarung durch die Schritte gelöst: Vorzugsweise Anordnen einer Umgebungskamera an einem beweglichen Mikroskopkopf eines Operationsmikroskops; Anvisieren eines Bereichs über ein in dem beweglichen Mikroskopkopf angeordnetes optisches System; Erstellen einer Mikroskopaufnahme durch eine an bzw. in dem Mikroskopkopf angeordneten Mikroskopaufnahmeeinheit einer durch das optische System bereitgestellten Vergrößerung; Erstellen einer Umgebungsaufnahme durch eine an dem Mikroskopkopf angeordneten Umgebungskamera, wobei ein Sichtfeld der Umgebungskamera, insbesondere ab einer vorbestimmen Entfernung, ein Sichtfeld des optischen Systems mit einschließt; Erstellen einer Kombinationsdarstellung mit der Mikroskopaufnahme und der Umgebungsaufnahme; Ausgeben der Kombinationsdarstellung durch eine Darstellungsvorrichtung. Durch das Darstellungsverfahren werden dem Chirurgen zentral beide Aufnahmemodalitäten zur Verfügung gestellt.

Das Darstellungsverfahren weist ferner die folgenden Schritte auf: Einlesen von präoperativen 3D-Aufnahmedaten, insbesondere MRT und/oder CT-Aufnahmedaten; Erfassen einer 3D-Struktur eines Patienten durch die Umgebungskamera; Korrelieren der erfassten 3D-Struktur mit den 3D-Aufnahmedaten; und Registrieren des Patienten über die Korrelation.

Gemäß einer Ausführungsform kann das Darstellungsverfahren die Schritte aufweisen: Initiale Registrierung des Patienten; Erfassen einer Lage des Mikroskopkopfs; Erstellen einer Umgebungsaufnahme durch die Umgebungskamera; Speichern der erfassten Lage (Registrierungsposition) und Umgebungsaufnahme; Bewegen des Mikroskopkopfs und anschließende Rückführung in die gespeicherte Lage; Erneute Umgebungsaufnahme; Vergleich der gespeicherten Umgebungsaufnahme mit der erneuten Umgebungsaufnahme; Bestimmen einer Abweichung zwischen der gespeicherten und der erneuten Umgebungsaufnahmen; Ausgabe eines akustischen oder visuellen Signals, wenn die Abweichung einen Grenzwert überschreitet und/oder Berechnung eines Offsets und Anwendung des Offsets auf die initiale Registrierung.

Insbesondere kann auch, wie vorstehend zu dem chirurgischen Assistenzsystem beschrieben und analog auf das Verfahren anwendbar, kontinuierlich eine Position der beobachteten Landmarken mit einer Referenzposition der Landmarken verglichen und eine etwaige Abweichung bestimmt werden. Bei Feststellen einer Abweichung kann insbesondere bestimmt werden, dass eine Abweichung einer Registrierung vorliegt und vorzugsweise eine Meldung ausgegeben werden. Bei Verwendung eines Verfahrens mit kontinuierlicher Erkennung einer Registrierungsabweichung kann etwa das Speichern der Registrierungsposition, die Aufnahme der Umgebung in der Registrierungsposition und das erneute Rückführen in die Registrierungsposition mit Aufnahme der Umgebung entfallen.

Gemäß einer weiteren Ausführungsform kann das Darstellungsverfahren die Schritte aufweisen: Erfassen eines medizinischen Instruments und dessen Position und/oder Orientierung in der Umgebungsaufnahme; Übertragen der Lage des medizinischen Instruments in 3D-Aufnahmedaten und Darstellung eines virtuellen medizinischen Instruments oder zumindest einer schematischen Ansicht in den 3D-Aufnahmedaten;
Vorzugsweise kann das Darstellungsverfahren die Schritte aufweisen:
Tracken eines Patienten und/oder eines chirurgischen Instruments und/oder des Mikroskopkopfs durch die Umgebungskamera und Bereitstellen der entsprechenden Bewegungsdaten.

Weiter kann das Darstellungsverfahren insbesondere die Schritte aufweisen: Bestimmen einer Distanz zu einem in der Umgebungsaufnahme erfassten Objekt; bei Unterschreitung einer Distanz: Ausgeben eines Alarmsignals und/oder Einschränkung von Bewegungsfreiheitsgraden des Mikroskopkopfs, insbesondere stoppen/sperren einer Bewegung des Mikroskopkopfs. Über die Umgebungsaufnahme der Umgebungskamera/Übersichtskamera können auch Kollisionen bei autonomen oder kollaborativen Bewegen des Mikroskoparms, insbesondere in Form eines Roboterarms, detektiert werden. Die Bewegung kann dann rechtzeitig durch ein Bremssystem gestoppt werden. Auch wenn der Mikroskoparm bzw. Roboterarm also manuell durch etwa den Chirurgen geführt wird, kann eine Kollision erkannt und eine weitere Bewegung unterbunden werden.

Gemäß einem Aspekt der Offenbarung kann das Darstellungsverfahren die Schritte aufweisen: Einlesen eines Fokuspunkts in der Umgebungsaufnahme; Steuern eines Mikroskoparms und des Mikroskopkopfs derart, dass die optische Mikroskopachse, insbesondere in einem vorbestimmen Abstand und/oder in einem bestimmen Aufnahmewinkel, auf den gewählten Fokuspunkt ausgerichtet wird.

Vorzugsweise kann das Darstellungsverfahren die Schritte aufweisen: Detektieren eines medizinischen Instruments in der Umgebungsaufnahme, Ausgeben von dem medizinischen Instrument zugehörigen Benutzungshinweisen über die Darstellungsvorrichtung.

An dieser Stelle wird darauf hingewiesen, dass Merkmale des Darstellungsverfahrens der vorliegenden Offenbarung sich sowohl auf das chirurgische Assistenzsystem der vorliegenden Offenbarung als auch umgekehrt übertragen lassen.

Hinsichtlich eines computerlesbaren Speichermediums werden die Aufgaben und Ziele der vorliegenden Offenbarung dadurch gelöst, dass das computerlesbare Speichermedium Befehle umfasst, die bei einer Ausführung durch einen Computer diesen veranlassen, die Verfahrensschritte des Aufnahmedarstellungsverfahren gemäß der vorliegenden Offenbarung auszuführen.

Hinsichtlich eines gattungsgemäßen Sterilraums wird die Aufgabe der vorliegenden Offenbarung dadurch gelöst, dass der medizinische Sterilraum ein chirurgisches Assistenzsystem gemäß der vorliegenden Offenbarung aufweist.

### Kurzbeschreibung der Figuren

Die vorliegende Offenbarung wird nachfolgend anhand bevorzugter Ausführungsformen mit Hilfe von begleitenden Figuren erläutert. Es zeigen:
- Fig. 1: eine schematische perspektivische Ansicht eines chirurgischen Assistenzsystems einer ersten bevorzugten Ausführungsform, bei der das chirurgische Assistenzsystem als mobiles Operationsmikroskop vorgesehen ist,
- Fig. 2: eine schematische perspektivische Ansicht eines Mikroskopkopfs des Operationsmikroskops aus Fig. 1,
- Fig. 3: eine weitere schematische perspektivische Ansicht des Assistenzsystems aus Fig. 1 und 2 während eines Eingriffs,
- Fig. 4: eine schematische Ansicht eines chirurgischen Assistenzsystems gemäß einer weiteren bevorzugten Ausführungsform mit einem Head-Mount-Display,
- Fig. 5: eine schematische Ansicht einer Erfassung einer 3D-Struktur durch die Umgebungskamera, und
- Fig. 6: ein Flussdiagramm eines Aufnahmedarstellungsverfahrens gemäß einer bevorzugten Ausführungsform.

Die Figuren sind lediglich schematischer Natur und sollen nur dem Verständnis der Offenbarung dienen. Gleiche Elemente sind mit denselben Bezugszeichen versehen. Die Merkmale der verschiedenen Ausführungsformen können untereinander ausgetauscht werden.

### Detaillierte Beschreibung bevorzugter Ausführungsformen.

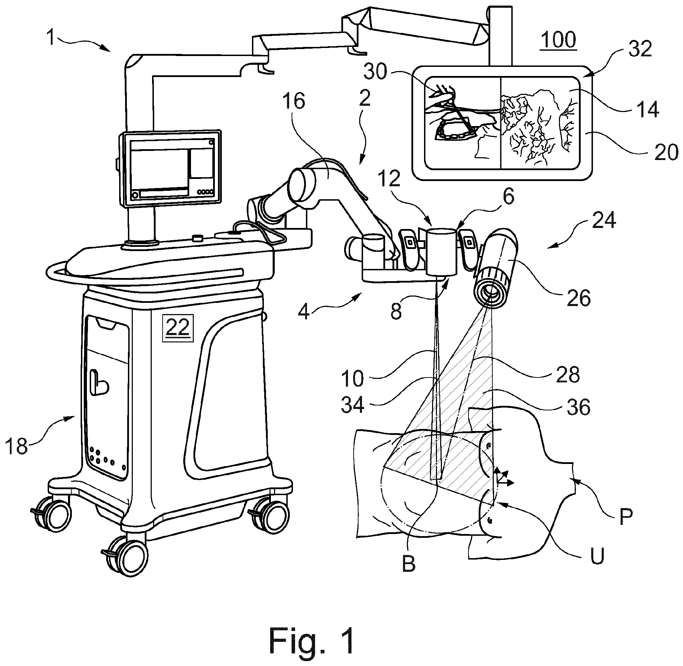

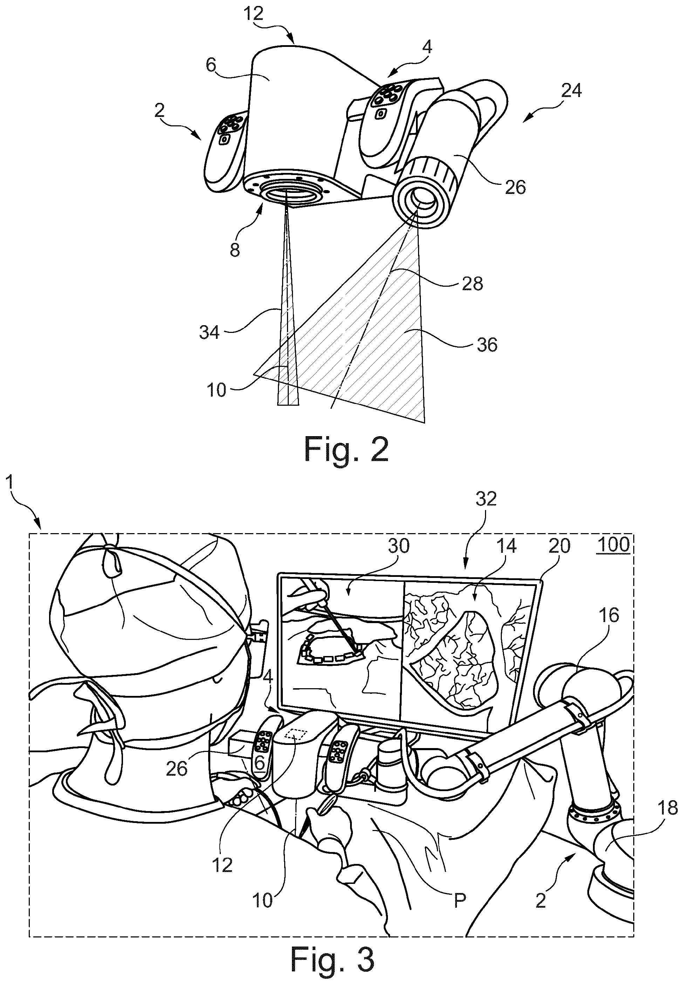

Figuren 1 bis 3 zeigen jeweils eine schematische perspektivische Ansicht eines chirurgisches Assistenzsystems 1 (nachstehend nur Assistenzsystem genannt) einer ersten bevorzugten Ausführungsform zur Verwendung bei einem chirurgischen Eingriff bei einem Patienten P. Das Assistenzsystem 1 kommt in einem medizinischen Sterilraum in Form eines Operationssaals 100 einer bevorzugten Ausführungsform zum Einsatz, um OP-Beteiligte, insbesondere einen Chirurgen, durch geeignete Visualisierung bei einem chirurgischen Eingriff bei einem Patienten P (hier nur schematisch dargestellt) zu unterstützen. In einem mittigen, sterilen, chirurgischen Eingriffsbereich wird an dem Patienten P ein minimalinvasiver Eingriff vorgenommen.

In dieser bevorzugten Ausführungsform weist das chirurgische Assistenzsystem 1 ein Operationsmikroskop 2 auf und ist sogar als mobiles, autark arbeitendes Operationsmikroskop 2 konfiguriert. Konkret weist das Operationsmikroskop 2 (nachstehend nur Mikroskop genannt) einen stirnseitigen beweglichen/justierbaren Mikroskopkopf 4 auf, der beweglich gelagert ist. So kann eine Lage (Position und Orientierung) des Mikroskopkopfs 4 individuell eingestellt werden.

Der bewegliche Mikroskopkopf 4 hat ein Gehäuse 6 und ein in diesem Gehäuse angeordnetes optisches System 8 mit einer Vielzahl an optischen Elementen wie etwa Linsen bzw. Linsensystemen. In dieser Ausführungsform ist ein monokulares optisches System vorgesehen. Es kann natürlich jedoch auch ein binokulares optisches System 8 vorgesehen werden, um etwa 3D-Mikroskopaufnahmen zu erstellen. Das optische System ist dafür angepasst in Richtung einer optischen Mikroskopachse 10 eine optische Vergrößerung, wie sie bei Operationsmikroskopen üblich ist, eines anvisierten Bereichs B, bereitzustellen. Insbesondere ist das optische System dafür angepasst eine Vergrößerung des anvisierten Bereichs B, der gegenüber einer Frontlinse (nicht dargestellt) in einer Entfernung zwischen 5cm bis 80cm liegt, scharf zu fokussieren und bereitzustellen. Diese bereitgestellte Vergrößerung wird mittels einer nachgeschalteten Mikroskopaufnahmeeinheit 12 (nachstehend nur Aufnahmeeinheit genannt), die einen Sensor aufweist, etwa eine CMOS Sensor oder einen CCD Sensor, erfasst bzw. aufgenommen und die Aufnahmeeinheit erstellt eine digitale/computerlesbare Mikroskopaufnahme 14.

Der Mikroskopkopf 4 ist an einem mit einer Basis 18 in Form eines Rollwagens gelagerten beweglichen Mikroskoparm 16 ebenfalls gelagert. Der Mikroskoparm 16 in Form eines, insbesondere kollaborativen, Roboterarms mit zwei Armelementen mit entsprechender Lagerung der Basis 18 und des Mikroskopkopfs 4 befähigt das Mikroskop 2 eine Position und/oder Orientierung des Mikroskopkopfes 4 im Raum einzustellen und auf einen anzuvisierenden Bereich B auszurichten, den der Chirurg vergrößert sehen will.

Um diese Vergrößerung auch visuell darzustellen, hat das Assistenzsystem 1 bzw. das mobile Mikroskop 2 einen OP-Monitor 20 an einem Ausleger. Eine Steuereinheit 22 verarbeitet die Mikroskopaufnahme 14 und gibt die Darstellung über den OP-Monitor 20 aus.

Im Unterschied zu bekannten Assistenzsystemen weist der Mikroskopkopf 4 des Mikroskops 2 zusätzlich ein Kamerasystem 24 mit einer Umgebungskamera 26 (nachstehend nur Kamera genannt) auf. Diese Kamera 26 ist an dem Gehäuse 6 des Mikroskopkopfs 4 starr befestigt so dass das optische System 8 des Mikroskops 2 und die Kamera 26 stets eine gleiche relative Ausrichtung zueinander aufweisen. Konkret ist die Umgebungskamera 26 über eine Ausrichtung ihrer optischen Kameraachse 28 und über ihre Kameraoptik dafür angepasst ist, dass ein (Kamera-)Sichtfeld 36 der Umgebungskamera 26 ab einer vorbestimmten Entfernung ein (Mikroskop-)Sichtfeld 34 des optischen Systems 8 mit einschließt, um durch die Umgebungskamera 26 sowohl den von dem optischen System 8 anvisierten Bereich B als auch eine Umgebung U um den anvisierten Bereich B herum zu erfassen und eine entsprechende Umgebungsaufnahme 30 durch die Kamera 26 bereitzustellen.

Die Steuervorrichtung 22 ist dafür angepasst, diese Umgebungsaufnahme 30 zu verarbeiten und eine Kombinationsdarstellung 32 in Form einer
Nebeneinanderdarstellung mit der Mikroskopaufnahme 14 und der Umgebungsaufnahme 30 zu erzeugen und durch die Darstellungsvorrichtung 20 visuell auszugeben.

Der bewegliche Mikroskopkopf 4 ist also mit einer zusätzlichen Umgebungskamera 26 ausgestattet. Die Blickrichtung bzw. die Ausrichtung der optischen Kameraachse 28 der Kamera 26 ist dabei ähnlich der Blickrichtung bzw. der optischen Mikroskopachse 10 des optischen Systems 8 des Mikroskops 2. Diese zusätzliche Kamera 26 erzeugt eine Weitwinkelansicht, die es ermöglicht, die Umgebung U des Operationsfeldes um einen durch das optische System 8 anvisierten Bereich B zu erfassen, insbesondere um Schnittgrenzen, Hände des Chirurgen oder verwendeten Instrumente zu erfassen. Die Darstellung des Bildes/der Aufnahme der Kamera 26 zusammen mit der Mikroskopaufnahme ermöglicht es dem Chirurgen, sowohl "das Innere" als auch "das Äußere" des Eingriffsbereichs zu sehen, ohne seine Kopfposition oder Blickrichtung zu verändern. Die Kamera 26 ist dabei in fester Relation zum Mikroskopkopf 4 montiert und ist dafür angepasst, die Umgebung U des Eingriffsbereichs auch bei geringen Abständen zwischen dem Mikroskopkopf 4 und dem Patienten P von beispielsweise 10-30 cm abzubilden bzw. zu erfassen. Der Chirurg sieht mit dem Assistenzsystem 1 sowohl die Mikroskopaufnahme 14 mit hoher Vergrößerung als auch die zusätzliche Umgebungsaufnahme 30 mit niedriger Vergrößerung auf dem OP-Monitor 20. So kann der Chirurg mit einem Blick intuitiv sowohl einen inneren hochauflösenden Bereich als auch einen übersichtlichen äußeren Bereich des Eingriffsbereichs sehen.

Der Mikroskopkopf 4 weist also zwei unterschiedliche "optische Systeme", nämlich das optische System 8 des Mikroskops 2 und eine Optik der Kamera 26 auf, die durch ihre Anordnung zueinander korreliert sind. Das optische System 8 des Mikroskops 2 dient zur Erzeugung eines Bildes mit hoher Vergrößerung und die zusätzliche Kamera 26 mit zugehöriger Optik zur Erzeugung eines Bildes mit Weitwinkel. Beide Optiken sind korreliert und die Blickrichtungen sind ähnlich und können auf den Eingriffsbereich/das Operationsfeld ausgerichtet werden. Das Kamerasystem 24 mit der Kamera 26 hat eine niedrige Vergrößerung und ein weites Sichtfeld 36, um eine Operationsumgebung mit etwa einer Schnittkante oder einer Hand und/oder einem Instrument um die Eingriffsstelle herum zu erfassen, während das optische System 8 eine hohe Vergrößerung mit geringerem Sichtfeld 34 hat. Die Kombinationsdarstellung 32 gibt dann schließlich die beiden Aufnahmen 14, 30 zentral wieder und ermöglicht dem Chirurgen als auch weiterem medizinischen Personal auf einen Blick beide Aufnahmemodalitäten korreliert zueinander zu sehen.

Das Kamerasystem 24 kann neben der Kamera 26 auch noch eine weitere Kamera aufweisen, die an dem Mikroskopkopf angebracht ist, um etwa eine Stereo-Aufnahme zu erstellen oder mehrere Ansichten aufzunehmen und in der Kombinationsdarstellung 32 entsprechend zusammenzuführen. Auch kann die Kamera 26 selbst neben einer 2D-Kamera auch eine 3D-Kamera bzw. Stereokamera sein.

Die Kamera 26 verfügt über eine Autofokusfunktion, um den anvisierten Bereich B bzw. die Umgebung scharf zu stellen. Auch kann die Kamera 26 über eine ZoomFunktion verfügen, um eine Umgebungsaufnahme 30 an einen jeweiligen Eingriff optimal anzupassen. Zusätzlich kann auch das optische System 8 eine Zoomfunktion aufweisen, um eine Vergrößerung des Mikroskops 2 anzupassen. Die Zoomfunktion kann kontinuierlich über sich relativ zueinander bewegende Linsen als auch diskret in Art eines Objektivrevolvers erfolgen. Insbesondere kann der Chirurg über einen Knopf am Mikroskopkopf ein Zoom/eine Vergrößerung einstellen. Auch kann die Kamera 26 und/oder das optische System 8 über eine einstellbare Blende verfügen, um eine Schärfe und einen Lichteinlass zu steuern.

Da die Steuereinheit 22 die beiden Aufnahmen digital verarbeitet, kann diese unterschiedliche digitale Analysen, Bildverarbeitungen und Steuerungen ausführen, insbesondere um eine passende Kombinationsdarstellung 32 zu erzeugen. Beispielsweise kann die Steuereinheit 22 dafür angepasst sein, verschiedene Bildparameter, wie Helligkeit oder Kontrast in den Aufnahmen zu ändern. Auch können weitere digitale Daten in die Kombinationsdarstellung 32 miteingefügt werden. Die Steuereinheit 22 kann dafür eine zentrale Prozessoreinheit (CPU), einen flüchtigen Speicher wie etwa RAM, ROM und einen nichtflüchtigen Speicher wie SSD aufweisen.

Der Mikroskoparm 16 ist über eine Vielzahl an Aktuatoren aktiv steuerbar. **In** den einzelnen Elementen des Mikroskoparms 16 vorgesehene Sensoren ermöglichen eine Bestimmung einer Lage des Mikroskoparms 16 sowie des Mikroskopkopfs 4, die an die Steuereinheit 22 weitergegeben wird. Der Chirurg kann auf dem OP-Monitor 20, der als Touchscreen ausgebildet ist, in der Umgebungsaufnahme 30 einen Fokuspunkt mit einem Finger manuell auswählen. Dieser gesetzte Fokuspunkt wird dann an die Steuereinheit 22 weitergegen, die ihrerseits die Aktuatoren des Mikroskoparms 16 sowie die Lagerung des Mikroskopkopfs aktiv ansteuert und bewegt und auf Basis des gesetzten Fokuspunkts die optische Mikroskopachse 10 in einem vorbestimmten Abstand und in einem senkrechten Aufnahmewinkel auf den Fokuspunkt bei dem Patienten P ausgerichtet wird. Auf diese Weise wird der anvisierte Bereich B auf den Fokuspunkt gesetzt und der Chirurg kann einfach an dieser Stelle seinen Eingriff vornehmen oder eine Vergrößerte Aufnahme sich anzeigen lassen. Auch kann der Fokuspunkt gespeichert werden, um zwischen verschiedenen Ansichten etwa automatisch hin-und her zuschalten.

Zusätzlich ist die Steuereinheit 22 ferner dafür angepasst über eine Bildanalyse der Umgebungsaufnahme 30 ein Objekt zu erfassen sowie ferner eine Distanz zu dem Objekt zu berechnen. Alternativ oder zusätzlich können an dem Mikroskopkopf 4 auch Distanzsensoren vorgesehen sein, insbesondere mehrere Distanzsensoren in unterschiedliche Richtungen, um ein Objekt und eine Distanz zu bestimmen. Bei einer möglichen Kollision mit dem so erfassten Objekt in dem Bewegungsfeld gibt das Assistenzsystem 1 über ein Audiogerät (nicht dargestellt) ein akustisches Alarmsignal aus (Kollisionswarnung) und sperrt eine Bewegung des Mikroskoparms 16. Insbesondere wird eine Bewegung des Mikroskoparm 16 und des Mikroskopkopfs 4 komplett gestoppt (Kollisionsverhinderung). Damit wird eine Kollision mit dem Objekt verhindert.

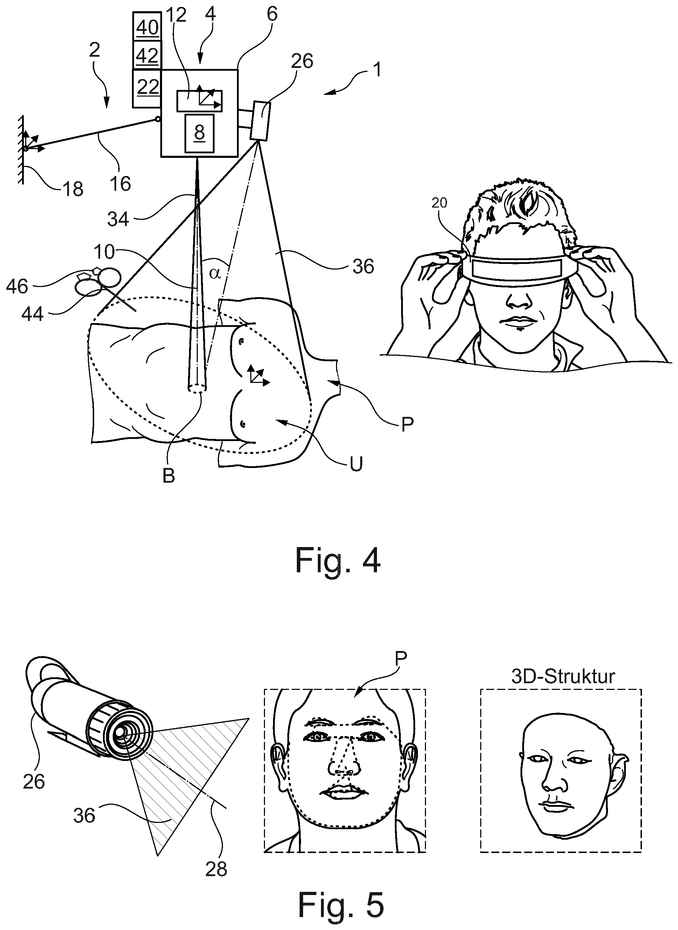

Fig. 4 zeigt ein chirurgisches Assistenzsystem 1 einer weiteren, zweiten bevorzugten Ausführungsform. Ähnlich zu der ersten Ausführungsform ist der Mikroskopkopf 4 über den Mikroskoparm 16 an einer festen Basis 18 beweglich gelagert und das optische System 8 kann einen Bereich B anvisieren. Die Umgebungskamera 26 ist dabei so ausgerichtet, dass die optische Kameraachse 28 die optische Mikroskopachse 10 schneidet und zwar im anvisierten Bereich B. Hierdurch wird gewährleistet, dass die Umgebungsaufnahme konzentrisch bzw. symmetrisch um den anvisierten Bereich herum erfolgt. Die Steuereinheit 22 verarbeitet wieder die beiden Aufnahmen 14, 30 und erstellt eine Kombinationsdarstellung 32.

Im Unterschied zu der der ersten Ausführungsform wird die Kombinationsdarstellung 32 jedoch nicht über einen Monitor, sondern über ein Head-Mount-Display/HMD-Brille 38 (nachstehend als Brille bezeichnet) ausgegeben. Die Brille 38 kann dafür ein Display und zusätzlich eine Optik aufweisen. Konkret steuert die Steuereinheit eine Sende-und Empfangseinheit 40 an, welche die Daten kabellos an die Brille 38 sendet. Dort werden die empfangenen Daten dann in der Brille 38 angezeigt.

Das Assistenzsystem 1 verwendet zudem ein chirurgisches Navigationssystem und weist ferner noch eine Speichereinheit 42 auf, in der eine MRT sowie eine CT-Aufnahme des Patienten P als 3D-Aufnahmedaten hinterlegt sind. Die Steuereinheit 22 ist dabei dafür angepasst, bei entsprechender Ausrichtung des Mikroskopkopfs 4 mit der Kamera 26 eine 3D-Struktur des Gesichts des Patienten (siehe Fig. 5) dreidimensional zu erfassen. Die so erfasste 3D-Struktur des Gesichts des Patienten wird mit der virtuellen 3D-Struktur des Gesichts des Patienten der 3D-Aufnahmedaten abgeglichen und die beiden 3D-Strukturen werden miteinander korreliert, um eine initiale Registrierung durchzuführen und den Patienten P gegenüber dem Mikroskopkopf 4 zu registrieren. Bei einer Bewegung des Mikroskopkopfs 4 erfassen Sensoren eine entsprechende Bewegung.

Fig. 5 zeigt schematisch eine solche Funktion einer Registrierung über 3D-Strukturen des Gesichts des Patienten, wie sie in dem Assistenzsystem in Fig. 4 vorgenommen wird. Die Kamera 26 ist eine 2D-Kamera, die über den interessierenden Bereich des Gesichts des Patienten bewegt wird (siehe gestrichelte Linien), um verschiedene Ansichten des Gesichts zu erhalten und über Machine-Vision-Methoden bzw. eine Bildanalyse, welche die Steuereinheit 22 ausführt, eine 3D-Struktur des Gesichts zu berechnen. Die so erhaltene 3D-Struktur des Gesichts kann gewissermaßen über die virtuelle 3D-Struktur der 3D-Aufnahmedaten gelegt bzw. in Wechselbeziehung gebracht werden oder die Steuereinheit 22 kann anhand der 3D-Struktur selbst eine Lage des Patienten P bestimmen.

Ferner ist die Steuereinheit 22 des Assistenzsystems 1 in Fig. 4 dafür angepasst, über an medizinischen Instrumenten 44 angebrachten Markern 46 oder charakteristische Merkmale, insbesondere geometrische Merkmale, ein solches medizinisches Instrument 44 über die Umgebungsaufnahme 30 zu erfassen und dessen Lage und/oder Funktion zu bestimmen. Im Falle der charakteristischen Merkmale kann in der Speichereinheit 42 eine virtuelle geometrische Relation des Instruments 44 gespeichert sein, welches die Steuereinheit 22 für einen Abgleich verwenden kann. Das so erfasste medizinische Instrument 44 kann dann in den 3D-Aufnahmedaten an der erfassten Lage eingeblendet und für eine chirurgische Navigation verwendet werden. Insbesondere kann die Steuereinheit in der Kombinationsdarstellung Daten zu Benutzungshinweisen zusätzlich einblenden, um den Chirurgen weitere Hilfestellungen bei seinem Eingriff bereitzustellen. Die Erfassung der Instrumente 44 mittels der Umgebungskamera 30 kann insbesondere dafür verwendet werden, dass die Steuereinheit 22 den Mikroskopkopf 4 mittels dem robotergeführten Mikroskoparm 16 optimal positioniert und/oder ausrichtet, um etwa ein gutes Sichtfeld auf den anvisierten Bereich B zu erhalten, ohne dass beispielsweise ein Instrument 44 das Sichtfeld verdeckt oder so positioniert und/oder ausrichtet, dass eine Instrumentenspitze noch im Sehfeld ist.

Die Steuereinheit 22 ist zudem dafür angepasst, automatisch eine Abweichung einer Navigation aufgrund von ungewollten Bewegungen von etwa eines Patiententrackers/Markers zu detektieren. Hierfür ist das Assistenzsystem 1 dafür angepasst, nach einer initialen Registrierung, den Mikroskopkopf 4 an eine vorbestimmte Lage (Registrierungsposition und -orientierung, nachstehend nur Registrierungsposition genannt) zu bewegen und über die Kamera 26 eine Umgebungsaufnahme 30 zu erstellen, die in der Speichereinheit 42 hinterlegt wird. Die Registrierungsposition wird als relative Transformation zwischen einem Patiententracker (nicht dargestellt) und einem Mikroskopkopftracker (nicht dargestellt) berechnet. Um nun sicherzustellen, dass keine Abweichung vorliegt, kann der Chirurg von Zeit zu Zeit während einer Operation den Mikroskopkopf 4 in die Registrierungsposition bewegen (lassen) und eine erneute Umgebungsaufnahme 30 anfertigen. Diese wird dann mit der in der Speichereinheit 42 hinterlegten Umgebungsaufnahme 30 überlagert und verglichen. Wenn keine oder eine geringe tolerierbare Abweichung vorliegt, so korrelieren Teilbereiche der beiden Umgebungsaufnahmen 30, die keiner Änderung unterliegen, wie etwa Schnittkanten oder Operationstücher, weiterhin miteinander und die Steuereinheit 22 bestimmt über eine entsprechende Bildanalyse, dass keine bzw. eine tolerierbare Abweichung vorliegt. Weichen jedoch Teilbereiche (mit eigentlich unveränderlichen Strukturen) der beiden Umgebungsaufnahmen 30 über einen tolerierbaren Wert/Grenzwert voneinander ab, so detektiert die Steuereinheit 22 dies und gibt eine Meldung an den Chirurgen über die Brille 38 oder einen Monitor aus. Auch berechnet die Steuereinheit 22 über geeignete Berechnungsmethoden ein Maß der Abweichung und gibt die Quantität der Abweichung dem Chirurgen aus. So kann der Chirurg bestimmen, ob diese Abweichung zu stark ist und entsprechende Maßnahmen ergreifen. Alternativ oder zusätzlich können in der Speichereinheit 42 der Steuereinheit 22 auch Bildverarbeitungsalgorithmen zur Realisierung einer kontinuierlichen Erkennung einer Registrierungsabweichung bzw. einer kontinuierlichen Verbesserung einer Genauigkeit der Registrierung hinterlegt sein.

Zusätzlich oder alternativ kann die Steuereinheit 22 auch dafür angepasst sein, einen Versatz/Offset zwischen der hinterlegten Umgebungsaufnahme 30 und der erneuten Umgebungsaufnahme 30 zu bestimmen und diesen Versatz auf die Registrierung bzw. auf eine Transformationsmatrix zwischen einem lokalen Koordinatensystem, etwa das des Mikroskopkopfs oder des chirurgischen Navigationssystems und dem lokalen Koordinatensystem des Patienten P entsprechend anzupassen und um diesen Versatz zu korrigieren.

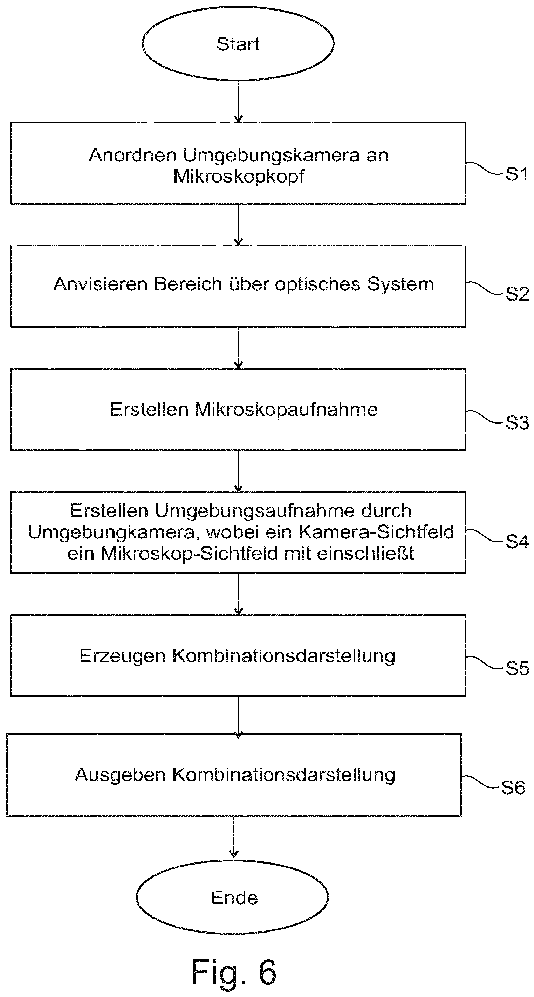

Fig. 6 zeigt ein Flussdiagramm eines Aufnahmedarstellungsverfahren (nachstehend nur Darstellungsverfahren genannt) gemäß einer bevorzugten Ausführungsform der vorliegenden Offenbarung zur korrelierten Anzeige von einer Mikroskopaufnahme 14 und einer Umgebungsaufnahme 30. Das Darstellungsverfahren kommt bei einem vorstehend beschriebenen chirurgischen Assistenzsystem 1 entsprechend zum Einsatz.

**In** einem ersten Schritt S1 kann vorzugsweise eine Umgebungskamera 26 an dem beweglichen Mikroskopkopf 4 des Operationsmikroskops 2 angeordnet, insbesondere starr befestigt werden.

**In** einem Schritt S2 wird ein Bereich B über das in dem beweglichen Mikroskopkopf 4 angeordnete optische System 8 anvisiert. **In** einem darauffolgenden Schritt S3 wird die Mikroskopaufnahme 14 durch die in dem Mikroskopkopf 4 angeordnete Mikroskopaufnahmeeinheit 12 der durch das optische System 8 bereitgestellten Vergrößerung erstellt. Zudem wird in einem Schritt S4 eine Umgebungsaufnahme 30 durch die an dem Mikroskopkopf 4 angeordneten Umgebungskamera 26 erstellt, wobei ein Sichtfeld 36 der Umgebungskamera 26, insbesondere ab einer vorbestimmen Entfernung, ein Sichtfeld 34 des optischen Systems 8 mit einschließt.

In einem Schritt S5 wird eine Kombinationsdarstellung 32 mit der Mikroskopaufnahme 14 und der Umgebungsaufnahme 30 durch die Steuereinheit 22 erstellt und die Kombinationsdarstellung 32 schließlich in einem Schritt S6 durch eine Darstellungsvorrichtung 20; 38 ausgegeben.

### Bezugszeichenliste

- 1: Chirurgisches Assistenzsystem
- 2: Operationsmikroskop
- 4: Mikroskopkopf
- 6: Gehäuse
- 8: Optisches System
- 10: Optische Mikroskopachse
- 12: Mikroskopaufnahmeeinheit
- 14: Mikroskopaufnahme
- 16: Mikroskoparm
- 18: Basis
- 20: OP-Monitor
- 22: Steuereinheit
- 24: Kamerasystem
- 26: Umgebungskamera
- 28: optische Kameraachse
- 30: Umgebungsaufnahme
- 32: Kombinationsdarstellung
- 34: Sichtfeld optisches System
- 36: Sichtfeld Kamera
- 38: Head-Mount-Display/Brille
- 40: Sende-und-Empfangseinheit
- 42: Speichereinheit
- 44: Medizinisches Instrument
- 46: Marker

- 100: Operationssaal

- P: Patient
- B: Anvisierter Bereich
- U: Umgebung
- S1: Schritt Anordnen einer Umgebungskamera
- S2: Schritt Anvisieren eines Bereichs
- S3: Schritt Erstellen einer Mikroskopaufnahme
- S4: Schritt Erstellen einer Umgebungsaufnahme
- S5: Schritt Erstellen einer Kombinationsdarstellung
- S6: Schritt Ausgeben Kombinationsdarstellung

## Patentansprüche

1. Chirurgisches Assistenzsystem (1) zur Verwendung bei einem chirurgischen Eingriff bei einem Patienten (P) mit:
einem Operationsmikroskop (2) mit:
- einem beweglichen Mikroskopkopf (4) aufweisend ein Gehäuse (6) und ein optisches System (8), das dafür angepasst ist, in Richtung einer optischen Mikroskopachse (10) eine optische Vergrößerung eines anvisierten Bereichs (B) bereitzustellen und mittels einer nachgeschalteten Mikroskopaufnahmeeinheit (12) eine digitale Mikroskopaufnahme (14) zu erstellen,
- einem mit einer Basis (18) verbundenen beweglichen Mikroskoparm (16), an dem der bewegliche Mikroskopkopf (4) angebunden, insbesondere gelagert, ist, und der dafür angepasst ist, eine Position und/oder Orientierung des Mikroskopkopfes (4) einzustellen;
zumindest einer Darstellungsvorrichtung (20; 38) zur Darstellung eines visuellen Inhalts; und
einer Steuereinheit (22), die dafür angepasst ist, die Mikroskopaufnahme (14) zu verarbeiten und die Darstellungsvorrichtung (20; 38) für eine Darstellung entsprechend anzusteuern,
wobei
das Operationsmikroskop (2), insbesondere der Mikroskopkopf (4), ferner ein Kamerasystem (24) mit zumindest einer Umgebungskamera (26), die an dem Mikroskopkopf (4), insbesondere an dem Gehäuse (6) und vorzugsweise starr, angeordnet ist, aufweist und die Umgebungskamera (26) dafür angepasst ist, insbesondere über eine Ausrichtung ihrer optischen Kameraachse (28) und über ihre Kameraoptik, dass ein Sichtfeld (36) der Umgebungskamera (26), insbesondere ab einer vorbestimmten Entfernung, ein Sichtfeld (34) des optischen Systems (8) mit einschließt, um durch die Umgebungskamera (26) sowohl den von dem optischen System (8) anvisierten Bereich (B) als auch eine Umgebung (U) um den anvisierten Bereich (B) herum zu erfassen und eine Umgebungsaufnahme (30) bereitzustellen, und
wobei die Steuereinheit (22) ferner dafür angepasst ist, die Umgebungsaufnahme (30) zu verarbeiten und eine Kombinationsdarstellung (32) mit der Mikroskopaufnahme (14) und der Umgebungsaufnahme (30) zu erzeugen und durch die Darstellungsvorrichtung (20; 38) visuell auszugeben, wobei das chirurgische Assistenzsystem (1) ferner eine Speichereinheit (42) mit präoperativen 3D-Aufnahmedaten, insbesondere MRT-Aufnahmedaten und/oder CT-Aufnahmedaten, aufweist und das Assistenzsystem (1), insbesondere die Steuereinheit (22), dafür angepasst ist, mittels dem Kamerasystem (24), insbesondere mit nur der Umgebungskamera (26), eine 3D-Struktur des Patienten (P), insbesondere eines Gesichts des Patienten (P), räumlich zu erfassen und die erfasste 3D-Struktur mit den 3D-Aufnahmedaten zu korrelieren, um den Patienten (P) zu registrieren.

2. Chirurgisches Assistenzsystem (1) nach Anspruch 1, **dadurch gekennzeichnet, dass** zur Erfassung der 3D-Struktur das Kamerasystem (24) eine 3D-Kamera und/oder eine 2D-Kamera aufweist, insbesondere aus der Umgebungskamera (26) als einzige 2D-Kamera oder 3D-Kamera des Kamerasystems (24) besteht, wobei im Falle der 2D-Kamera diese über einen interessierenden Bereich des Patienten (P) bewegt wird, um verschiedene Ansichten des interessierenden Bereichs zu erhalten, und die Steuereinheit (22) dafür angepasst ist, mittels Bildanalyse aus den verschiedenen Ansichten die 3D-Struktur zu berechnen.

3. Chirurgisches Assistenzsystem (1) nach einem der vorstehenden Ansprüche , **dadurch gekennzeichnet, dass** das Assistenzsystem (1) dafür ausgebildet ist, eine Position, insbesondere Lage, des Mikroskopkopfs (4), zu erfassen und der Steuereinheit (22) bereitzustellen, und die Steuereinheit (22) dafür angepasst ist, die Position, insbesondere die Lage, des Mikroskopkopfs (4) in einer Speichereinheit (42) als Registrierungsposition sowie die zugehörige Umgebungsaufnahme (30), insbesondere eine erfasste 3D-Struktur, zu speichern, und bei erneuter Umgebungsaufnahme (30) in der hinterlegten Registrierungsposition bzw. Lage, die Steuereinheit (22) bestimmt, dass eine Abweichung vorliegt, wenn eine Überlagerung, insbesondere eine Teilbereichsüberlagerung, der hinterlegten Umgebungsaufnahme (30) und der erneut erfassten Umgebungsaufnahme (30) über einen vorbestimmten Grenzwert hinaus voneinander abweichen.

4. Chirurgisches Assistenzsystem (1) nach einem der vorstehenden Ansprüche, **dadurch gekennzeichnet, dass** das Operationsmikroskop (2) zumindest einen Aktuator aufweist, um den beweglichen Mikroskoparm (16) und den beweglichen Mikroskopkopf (4) aktiv zu bewegen und die Steuereinheit (22) ferner dafür angepasst ist den Aktuator anzusteuern, um den Mikroskopkopf (4) aktiv in eine vorbestimmte Position und/oder Orientierung zu bewegen.

5. Chirurgisches Assistenzsystem (1) nach Anspruch 4, **dadurch gekennzeichnet, dass** das Assistenzsystem (1) eine Eingabeeinheit, insbesondere einen Touchscreen, aufweist, um in der Umgebungsaufnahme (30) einen Fokuspunkt auszuwählen, und die Steuereinheit (22) dafür angepasst ist, auf Basis des gewählten Fokuspunkts über den zumindest einen Aktuator aktiv den Mikroskoparm (16) und den Mikroskopkopf (4) so zu steuern, dass die optische Mikroskopachse (10), insbesondere in einem vorbestimmten zugehörigen Abstand und/oder einem vorbestimmten Aufnahmewinkel, auf den ausgewählten Fokuspunkt bei dem Patienten (P) ausgerichtet ist.

6. Chirurgisches Assistenzsystem (1) nach einem der vorstehenden Ansprüche, **dadurch gekennzeichnet, dass** die Steuereinheit (22) dafür angepasst ist, mittels Bildanalyse der Umgebungsaufnahme (30) und/oder einem an dem Mikroskopkopf (4) vorgesehenen Sensor, ein Objekt im Bereich des Mikroskopkopfs (4) sowie eine Distanz des beweglichen Mikroskopkopfs (4) zu diesem Objekt zu erkennen und bei einer möglichen Kollision mit diesem Objekt, insbesondere bei Unterschreitung einer vorbestimmten Distanz, ein Alarmsignal auszugeben und/oder zumindest einen Bewegungsfreiheitsgrad des Mikroskopkopfs (4) zu beschränken, insbesondere insgesamt eine Bewegung des Mikroskopkopfs (4) zu stoppen.

7. Chirurgisches Assistenzsystem (1) nach einem der vorstehenden Ansprüche, **dadurch gekennzeichnet, dass** die Steuereinheit (22) dafür angepasst ist, über das Kamerasystem (24), insbesondere über zumindest die Umgebungsaufnahme (30), insbesondere über Marker und/oder über charakteristische Merkmale des Patienten (P), den Patienten (P) und/oder, insbesondere über Marker (46) und/oder charakteristische Merkmale von medizinischen Instrumenten (44), ein medizinisches Instrument (44) und/oder den Mikroskopkopf (4) lagekorrekt zu erfassen, insbesondere kontinuierlich zu tracken.

8. Chirurgisches Assistenzsystem (1) nach einem der vorstehenden Ansprüche, **dadurch gekennzeichnet, dass** in einer Speichereinheit (42) Daten, insbesondere geometrische Relationen, von zumindest einem medizinischen Instrument (44) und zugehörige Benutzungshinweise gespeichert sind und die Steuereinheit (22) dafür angepasst ist, in der Umgebungsaufnahme (30) anhand der gespeicherten Daten, insbesondere der geometrischen Relation, ein chirurgisches Instrument (44) zu erfassen und einem Nutzer die zugehörigen Benutzungshinweise über die Darstellungsvorrichtung (20; 38) auszugeben.

9. Chirurgisches Assistenzsystem (1) nach einem der vorstehenden Ansprüche, **dadurch gekennzeichnet, dass** die zumindest eine Darstellungsvorrichtung ein OP-Monitor (20) und/oder ein Head-Mount-Display (38) und/oder ein binokulares System mit einblendbaren Daten ist.

10. Chirurgisches Assistenzsystem (1) nach einem der vorstehenden Ansprüche, **dadurch gekennzeichnet, dass** eine Vergrößerung des optischen Systems (8) mindestens fünffach, insbesondere mindestens zehnfach ist und vorzugsweise das optische System (8) über eine Zoomfunktion verfügt und/oder eine Vergrößerung der Umgebungskamera (26) maximal fünffach ist, insbesondere maximal einfach.

11. Aufnahmedarstellungsverfahren zur korrelierten Anzeige von einer Mikroskopaufnahme (14) und einer Umgebungsaufnahme (30), insbesondere für ein chirurgisches Assistenzsystem (1) nach einem der Ansprüche 1 bis 10, mit den Schritten:
- (S2) Anvisieren eines Bereichs (B) über ein an einem beweglichen Mikroskopkopf (4) angeordnetes optisches System (8);
- (S3) Erstellen einer Mikroskopaufnahme (14) durch eine an dem Mikroskopkopf (4) angeordneten Mikroskopaufnahmeeinheit (12) einer durch das optische System (8) bereitgestellten Vergrößerung;
- (S4) Erstellen einer Umgebungsaufnahme (30) durch eine an dem Mikroskopkopf (4) angeordneten Umgebungskamera (26), wobei ein Sichtfeld (36) der Umgebungskamera (26), insbesondere ab einer vorbestimmten Entfernung, ein Sichtfeld (34) des optischen Systems (8) mit einschließt;
- (S5) Erstellen einer Kombinationsdarstellung (32) mit der Mikroskopaufnahme (14) und der Umgebungsaufnahme (30);
- (S6) Ausgeben der Kombinationsdarstellung (32) durch eine Darstellungsvorrichtung (20; 38);
- Einlesen von präoperativen 3D-Aufnahmedaten, insbesondere MRT und/oder CT-Aufnahmedaten;
- Erfassen einer 3D-Struktur eines Patienten (P) durch die Umgebungskamera (26);
- Korrelieren der erfassten 3D-Struktur mit den 3D-Aufnahmedaten; und
- Registrieren des Patienten (P) über die Korrelation.

12. Computerlesbares Speichermedium, umfassend Befehle, die bei einer Ausführung durch einen Computer diesen veranlassen, die Verfahrensschritte gemäß Anspruch 11 auszuführen.

13. Medizinischer Sterilraum, wie etwa ein Operationssaal (100), **dadurch gekennzeichnet, dass** der medizinische Sterilraum ein chirurgisches Assistenzsystem (1) nach einem der Ansprüche 1 bis 10 aufweist.

## Claims

1. A surgical assistance system (1) for use in a surgical intervention on a patient (P) with:
a surgical microscope (2) with:
- a movable microscope head (4) comprising a housing (6) and an optical system (8) adapted to provide optical magnification of a targeted area (B) in the direction of an optical microscope axis (10) and to create a digital microscope image (14) via a downstream microscope image unit (12),
- a movable microscope arm (16) connected to a base (18), on which the movable microscope head (4) is attached, in particular supported, and which is adapted to adjust a position and/or orientation of the microscope head (4);
at least one display device (20; 38) for displaying a visual content; and
a control unit (22) adapted to process the microscope image (14) and to control the display device (20; 38) for a view accordingly,
wherein the surgical microscope (2), in particular the microscope head (4), further comprises a camera system (24) with at least one surroundings camera (26), which is arranged on the microscope head (4), in particular on the housing (6) and preferably rigidly, and the surroundings camera (26) is adapted in such a way, in particular via an orientation of its optical camera axis (28) and via its camera optics, that a field of view (36) of the surroundings camera (26), in particular from a predetermined distance, includes a field of view (34) of the optical system (8), in order to detect, by the surroundings camera (26), both the area (B) targeted by the optical system (8) and surroundings (U) around the targeted area (B) and to provide a surroundings image (30), and
wherein the control unit (22) is further adapted to process the surroundings image (30) and to generate a combined view (32) with the microscope image (14) and the surroundings image (30) and to output it visually by the display device (20; 38), wherein the surgical assistance system (1) further comprises a storage unit (42) with preoperative 3D image data, in particular MRI image data and/or CT image data, and the assistance system (1), in particular the control unit (22), is adapted to spatially detect a 3D structure of the patient (P), in particular of a face of the patient (P), via the camera system (24), in particular with only the surroundings camera (26), and to correlate the detected 3D structure with the 3D image data in order to register the patient (P).

2. The surgical assistance system (1) according to claim 1, **characterized in that** for the detection of the 3D structure, the camera system (24) has a 3D camera and/or a 2D camera, in particular consisting of the surroundings camera (26) as the only 2D camera or 3D camera of the camera system (24), wherein in the case of the 2D camera, it is moved over an area of interest of the patient (P) in order to obtain different views of the area of interest, and the control unit (22) is adapted to compute the 3D structure from the different views using image analysis.

3. The surgical assistance system (1) according to one of the preceding claims, **characterized in that** the assistance system (1) is configured to detect a position, in particular a pose, of the microscope head (4) and to provide it to the control unit (22), and the control unit (22) is adapted to store the position, in particular the pose, of the microscope head (4) in a storage unit (42) as a registration position and the associated surroundings image (30), in particular a detected 3D structure, and, upon a renewed surroundings image (30) in the stored registration position or pose, the control unit (22) determines that a deviation exists if an overlay, in particular a partial area overlay, of the stored surroundings image (30) and the newly detected surroundings image (30) deviate beyond a predetermined threshold.

4. The surgical assistance system (1) according to one of the preceding claims, **characterized in that** the surgical microscope (2) comprises at least one actuator in order to actively move the movable microscope arm (16) and the movable microscope head (4), and the control unit (22) is further adapted to control the actuator to actively move the microscope head (4) to a predetermined position and/or orientation.

5. The surgical assistance system (1) according to claim 4, **characterized in that** the assistance system (1) comprises an input unit, in particular a touch screen, for selecting a focus point in the surroundings image (30), and the control unit (22) is adapted to actively control the microscope arm (16) and the microscope head (4) based on the selected focus point via the at least one actuator such that the optical microscope axis (10) is aligned with the selected focus point at the patient (P), in particular at a predetermined associated distance and/or a predetermined image angle.

6. The surgical assistance system (1) according to one of the preceding claims, **characterized in that** the control unit (22) is adapted to detect, via image analysis of the surroundings image (30) and/or a sensor provided at the microscope head (4), an object in the area of the microscope head (4) as well as a distance of the movable microscope head (4) to this object and, in the event of a possible collision with this object, in particular if the distance falls below a predetermined distance, to emit an alarm signal and/or to restrict at least a degree of freedom of movement of the microscope head (4), in particular to stop overall movement of the microscope head (4).

7. The surgical assistance system (1) according to one of the preceding claims, **characterized in that** the control unit (22) is adapted to use the camera system (24), in particular at least the surroundings image (30), in particular markers and/or characteristic features of the patient (P), to positionally correctly detect and in particular continuously track the patient (P) and/or a medical instrument (44) and/or the microscope head (4) using markers (46) and/or characteristic features of medical instruments (44).

8. The surgical assistance system (1) according to one of the preceding claims, **characterized in that** data, in particular geometric relations, of at least one medical instrument (44) and associated use instructions are stored in a storage unit (42), and the control unit (22) is adapted to detect a surgical instrument (44) in the surroundings image (30) based on the stored data, in particular based on the geometric relation, and to output the associated use instructions to a user via the display device (20; 38).

9. The surgical assistance system (1) according to one of the preceding claims, **characterized in that** the at least one display device is an OR monitor (20) and/or a head-mounted display (38) and/or a binocular system with data capable of fading in.

10. The surgical assistance system (1) according to one of the preceding claims, **characterized in that** a magnification of the optical system (8) is at least fivefold, in particular at least tenfold, and preferably the optical system (8) has a zoom function and/or a magnification of the surroundings camera (26) is at most fivefold, in particular at most single.

11. An image display method for correlated display of a microscope image (14) and a surroundings image (30), in particular for a surgical assistance system (1) according to one of claims 1 to 10, comprising the steps:
- (S2) targeting an area (B) via an optical system (8) arranged on a movable microscope head (4);
- (S3) creating a microscope image (14) by a microscope image unit (12) arranged on the microscope head (4) of a magnification provided by the optical system (8);
- (S4) creating a surroundings image (30) by a surroundings camera (26) arranged on the microscope head (4), wherein a field of view (36) of the surroundings camera (26), in particular from a predetermined distance, includes a field of view (34) of the optical system (8);
- (S5) creating a combined view (32) with the microscope image (14) and the surroundings image (30);
- (S6) outputting the combined view (32) by a display device (20; 38);
- reading in preoperative 3D image data, in particular MRI and/or CT image data;
- detecting a 3D structure of a patient (P) by the surroundings camera (26);
- correlating the detected 3D structure with the 3D image data; and
- registering the patient (P) based on the correlation.

12. A computer-readable storage medium comprising instructions which, when executed by a computer, cause the computer to perform the method steps of claim 12.

13. A medical sterile space, such as an operation room (100), **characterized in that** the medical sterile space comprises a surgical assistance system (1) according to one of claims 1 to 10.

## Revendications

1. Système d'assistance chirurgicale (1) pour utilisation lors d'une intervention chirurgicale auprès d'un patient (P), comprenant :
un microscope opératoire (2) équipé :
- d'une tête de microscope mobile (4) présentant un boîtier (6) et un système optique (8), qui est adapté pour fournir, dans la direction d'un axe optique de microscope (10) un grossissement optique d'une zone ciblée (B), et pour réaliser une micrographie numérique (14) au moyen d'une unité de capture d'images microscopiques commutée en aval (12),
- d'un bras de microscope mobile (16) relié à une base (18), auquel la tête de microscope mobile (4) est rattachée, en particulier montée sur palier, et qui est adapté pour régler une position et/ou une orientation de la tête de microscope (4) ;
d'au moins un dispositif d'affichage (20 ; 38) pour l'affichage d'un contenu visuel ; et
d'un dispositif de commande (22) adaptée pour traiter la micrographie (14) et pour commander de manière correspondante le dispositif d'affichage (20 ; 38) pour un affichage,
dans lequel le microscope opératoire (2), en particulier la tête de microscope (4), comporte en outre un système de caméra (24) doté d'au moins une caméra d'environnement (26) qui est disposée sur la tête de microscope (4), en particulier sur le boîtier (6) et de préférence de manière fixe, et la caméra d'environnement (26) est adaptée, en particulier par le biais d'un alignement de son axe de caméra optique (28) et par le biais de son optique de caméra, de sorte qu'un champ de vision (36) de la caméra d'environnement (26), en particulier à partir d'une distance prédéterminée, englobe un champ de vision (34) du système optique (8), afin de capturer par le biais de la caméra d'environnement (26) tant la zone ciblée (B) par le système optique (8) qu'un environnement (U) autour de la zone ciblée (B) et de fournir une prise de vue de l'environnement (30), et
dans lequel le dispositif de commande (22) est en outre adaptée pour traiter la prise de vue de l'environnement (30) et pour générer une représentation combinée (32) comprenant la micrographie (14) et la prise de vue de l'environnement (30) et pour la délivrer visuellement par le biais du dispositif d'affichage (20 ; 38), dans lequel le système d'assistance chirurgicale (1) comporte en outre une unité de mémoire (42) contenant des données d'images 3D préopératoires, en particulier des données d'images d'IRM et/ou des données d'images de TDM, et le système d'assistance (1), en particulier l'unité de commande (22), est adapté pour capturer spatialement, au moyen du système de caméra (24), en particulier avec la caméra d'environnement (26) uniquement, une structure 3D du patient (P), en particulier d'un visage du patient (P), et pour corréler la structure 3D capturée avec les données d'acquisition 3D afin d'enregistrer le patient (P).

2. Système d'assistance chirurgicale (1) selon la revendication 1, **caractérisé en ce que,** pour la capture de la structure 3D, le système de caméra (24) comporte une caméra 3D et/ou une caméra 2D, en particulier se compose de la caméra d'environnement (26) en tant qu'unique caméra 2D ou caméra 3D du système de caméra (24), dans lequel, dans le cas de la caméra 2D, celle-ci est déplacée au-dessus d'une zone d'intérêt du patient (P) afin d'obtenir différentes vues de la zone d'intérêt, et le dispositif de commande (22) est adaptée pour calculer la structure 3D à partir des différentes vues au moyen d'une analyse d'image.

3. Système d'assistance chirurgicale (1) selon l'une des revendications précédentes, **caractérisé en ce que** le système d'assistance (1) est configuré pour capturer une position, en particulier une orientation, de la tête de microscope (4) et pour la fournir à l'unité de commande (22), et l'unité de commande (22) est adaptée pour sauvegarder la position, en particulier l'orientation, de la tête de microscope (4) dans une unité de mémoire (42) en tant que position d'enregistrement ainsi que la prise de vue de l'environnement (30) associée, en particulier une structure 3D capturée, et lors d'une nouvelle prise de vue de l'environnement (30) dans la position ou l'orientation d'enregistrement mémorisée, le dispositif de commande (22) détermine qu'il existe un écart lorsqu'une superposition, en particulier une superposition de zones partielles, de la prise de vue de l'environnement (30) mémorisée et de la prise de vue de l'environnement (30) nouvellement capturée, présente un écart supérieur à une valeur seuil prédéterminée.

4. Système d'assistance chirurgicale (1) selon l'une quelconque des revendications précédentes, **caractérisé en ce que** le microscope opératoire (2) comporte au moins un actionneur pour déplacer activement le bras de microscope mobile (16) et la tête de microscope mobile (4), et le dispositif de commande (22) est en outre adaptée pour piloter l'actionneur afin de déplacer activement la tête de microscope (4) dans une position et/ou une orientation prédéterminée.

5. Système d'assistance chirurgicale (1) selon la revendication 4, **caractérisé en ce que** le système d'assistance (1) comporte une unité de saisie, en particulier un écran tactile, pour sélectionner un point focal dans la prise de vue de l'environnement (30), et le dispositif de commande (22) est adaptée pour commander activement, sur la base du point focal sélectionné et via le au moins un actionneur, le bras de microscope (16) et la tête de microscope (4), de telle sorte que l'axe optique du microscope (10) soit aligné sur le point focal sélectionné chez le patient (P), en particulier à une distance associée prédéterminée et/ou selon un angle de prise de vue prédéterminé.

6. Système d'assistance chirurgicale (1) selon l'une quelconque des revendications précédentes, **caractérisé en ce que** le dispositif de commande (22) est adaptée pour détecter, au moyen d'une analyse d'image de la prise de vue de l'environnement (30) et/ou d'un capteur prévu sur la tête de microscope (4), un objet présent dans le champ de la tête de microscope (4) ainsi qu'une distance de la tête de microscope mobile (4) par rapport à cet objet et, en cas de collision potentielle avec cet objet, notamment lorsque la distance est inférieure à une distance prédéterminée, pour émettre un signal d'alarme et/ou restreindre au moins un degré de liberté de mouvement de la tête de microscope (4), en particulier pour arrêter totalement un mouvement de la tête de microscope (4).

7. Système d'assistance chirurgicale (1) selon l'une quelconque des revendications précédentes, **caractérisé en ce que** le dispositif de commande (22) est configurée pour capturer en position réelle le patient (P) au moyen du système de caméra (24), notamment au moyen d'au moins la prise de vue de l'environnement (30), en particulier via des marqueurs et/ou des caractéristiques distinctives du patient (P), et/ou un instrument médical (44) et/ou la tête de microscope (4), notamment via des marqueurs (46) et/ou des caractéristiques distinctives d'instruments médicaux (44), et notamment pour en assurer un suivi continu.

8. Système d'assistance chirurgicale (1) selon l'une quelconque des revendications précédentes, **caractérisé en ce que** des données, en particulier des relations géométriques, d'au moins un instrument médical (44), ainsi que des consignes d'utilisation associées, sont mémorisées dans une unité de mémoire (42), et **en ce que** le dispositif de commande (22) est adaptée pour détecter un instrument chirurgical (44) dans la prise de vue de l'environnement (30) à l'aide des données mémorisées, en particulier de la relation géométrique, et pour fournir à un utilisateur les consignes d'utilisation associées par l'intermédiaire du dispositif d'affichage (20 ; 38).

9. Système d'assistance chirurgicale (1) selon l'une quelconque des revendications précédentes, **caractérisé en ce que** le au moins un dispositif d'affichage est un moniteur de bloc opératoire (20) et/ou un visiocasque (38) (Head-Mount-Display) et/ou un système binoculaire à affichage de données intégré.

10. Système d'assistance chirurgicale (1) selon l'une quelconque des revendications précédentes, **caractérisé en ce qu'**un grossissement du système optique (8) est d'au moins cinq fois, en particulier d'au moins dix fois, et de préférence le système optique (8) dispose d'une fonction zoom, et/ou un grossissement de la caméra d'environnement (26) est au maximum de cinq fois, notamment au maximum d'une fois.

11. Procédé de représentation d'images pour l'affichage corrélé d'une micrographie (14) et d'une prise de vue de l'environnement (30), en particulier pour un système d'assistance chirurgicale (1) selon l'une quelconque des revendications 1 à 10, comprenant les étapes suivantes :
- (S2) Visée d'une zone (B) par l'intermédiaire d'un système optique (8) disposé sur une tête de microscope mobile (4) ;
- (S3) Acquisition d'une micrographie (14), par une unité de capture d'images microscopiques (12) agencée sur la tête de microscope (4), d'un grossissement fourni par le système optique (8) ;
- (S4) Acquisition d'une prise de vue de l'environnement (30) par une caméra d'environnement (26) agencée sur la tête de microscope (4), dans laquelle un champ de vision (36) de la caméra d'environnement (26) englobe, en particulier à partir d'une distance prédéterminée, un champ de vision (34) du système optique (8) ;
- (S5) Acquisition d'une représentation combinée (32) comportant la micrographie (14) et la prise de vue de l'environnement (30) ;
- (S6) Affichage de la représentation combinée (32) par un dispositif d'affichage (20 ; 38) ;
- Lecture de données d'images 3D préopératoires, en particulier de données d'images d'IRM et/ou de TDM ;
- Détection d'une structure tridimensionnelle 3D du patient (P) par la caméra d'environnement (26) ;
- Corrélation de la structure 3D acquise avec les données d'images 3D ; et
- Enregistrement du patient (P) au moyen de la corrélation.

12. Support de stockage lisible par ordinateur, comprenant des ordres qui, lorsqu'ils sont exécutés par un ordinateur, amènent celui-ci à exécuter les étapes de procédé selon la revendication 11.

13. Salle stérile médicale, telle qu'une salle d'opération (100), **caractérisée en ce que** la salle stérile médicale présente un système d'assistance chirurgicale (1) selon l'une quelconque des revendications 1 à 10.
